# EUROPEAN PATENT APPLICATION

(11) **EP 2 289 430 A2**
(43) Date of publication of application: **02.03.2011**
(21) Application number: 10176333.2
(22) Date of filing: 11.01.2007
(51) Int. Cl.: A61B 17/11, A61B 17/22, A61B 17/00, A61B 19/00, A61M 25/09

(54) **Equipment to approximate tissue portions, which are intended to form an anastomosis, and a method for performing anastomoses in tracts of the digestive tube**

(30) Priority: 16.01.2006 IT MI20060058
(62) Divisional of application: 07702680.5
(71) Applicant: Ethicon Endo-Surgery, Inc., Cincinnati, Ohio 45242-2839 (US)
(72) Inventor: Tacchino, Roberto, I-00144, ROMA (IT); D'Arcangelo, Michele, I-00142, ROMA (IT); Kuhns, Jesse J., Cincinnati, OH 45208 (US); Pastorelli, Alessandro, I-00136, ROMA (IT); Bilotti, Federico, I-04011, Aprilia, LATINA (IT)
(74) Representative: Leihkauf, Steffen Falk

(57) **Abstract**

An equipment to approximate tissue portions, which are intended to form an anastomosis comprises a guide means suitable for passing through a first tissue portion (12) and a second tissue portion (14) to be connected by anastomosis. This equipment further comprises an anastomotic device (10) to approximate the first tissue portion (12) and the second tissue portion (14) to be connected by anastomosis. The guide means consists of at least two guide wires (A, B), suitable for passing through the tissue portions to be connected, to receive and drag at least said anastomotic device (10) to approximate the first tissue portion (12) and the second tissue portion (14) to be connected by anastomosis. The guide wires (A, B) are located side by side to each other and are suitable for forming a loop which passes through the tissue portions to be connected.

## Description

In accordance with a first aspect, it is an object of the present invention an equipment to approximate tissue portions, which are intended to form an anastomosis. The aforesaid equipment is particularly suitable for being applied endoluminally, even if it can be used in partially or wholly laparoscopic techniques or conventional surgical techniques.

The present invention further relates to a method for performing anastomoses in tracts of the digestive tube, particularly suitable for being performed endoluminally, even if a use is possible in partially or wholly laparoscopic techniques or in conventional surgery techniques.

The known equipments allow a limited control of the operations performed, in particular during the positioning of anastomotic devices suitable for approximating tissues and for performing anastomoses. Furthermore, the known equipments are not suitable for being applied in procedures at least partially of an endoluminal kind.

In addition to what set forth above, the known methods to perform anastomoses in tracts of the digestive tube are quite invasive, involve a long hospital stay and have a high degree of risk.

As is known, the endoluminal approach considerably minimize the drawbacks of the conventional surgical or laparoscopic methodology. In particular, it allows minimizing the invasiveness of the procedure, decreasing the risks for the patient and shortening the postoperative course.

It is currently extremely difficult to utilize the endoluminal approach to perform anastomoses in tracts of the digestive tube, in particular due to the lack of tools suitable to this aim. Consequently, the anastomoses of tracts of the digestive tube, for example to perform gastro-intestinal bypasses, are still conducted through conventional surgical or laparoscopic techniques.

The problem underlying the present invention is to propose an equipment to approximate tissue portions, which are intended to form an anastomosis, which allows to solve the drawbacks cited with reference to the prior art.

A further problem underlying the present invention is to provide an equipment to approximate tissue portions intended to form an anastomosis particularly suitable for the endoluminal use, thus allowing to meet the increasing need to broaden the utilization fields for this technique, while being suitable also for a use in partially or wholly laparoscopic techniques or in conventional surgery techniques.

This problem is solved by means of an equipment to approximate tissue portions intended to form an anastomosis according to claim 1.

A further problem underlying the present invention is to provide a method for performing anastomoses in tracts of the digestive tube which allows to solve the drawbacks cited with reference to the prior art, particularly but not necessarily a method performed at least partially endoluminally.

The aforesaid problem is solved through a method for performing anastomoses in tracts of the digestive tube according to claim 48.

Further features and advantages of the equipment and the method according to the invention will be understood from the description set forth herein below of preferred and exemplificative embodiments, given by way of illustration but not limitation, by reference to the attached Figs., in which:
Fig. 1 illustrates a partially sectioned perspective view of a portion of stomach and intestine;
Fig. 2 illustrates a step of the method for performing anastomoses in tracts of the digestive tube according to the present invention carried out on the portion of Fig. 1;
Figs. 3 to 6 illustrate perspective and enlarged views of a detail from Fig. 1, respectively corresponding to further steps of the method according to the present invention;
Fig. 7 illustrates the perspective and partially sectioned view of Fig. 1 at the end of a first sequence of steps of the method according to the present invention;
Fig. 8 illustrates the perspective and partially sectioned view of Fig. 7 according to a possible variation of the method according to the present invention;
Fig. 9 illustrates a further step of the method according to the present invention carried out on the portion of Fig. 7;
Figs. 10 and 11 illustrate perspective and enlarged views of a detail from Fig. 7, respectively corresponding to further steps of the method according to the present invention;
Fig. 12 illustrates a further step of the method according to the present invention carried out on the portion of Fig. 7;
Fig. 13 illustrates a perspective and enlarged view of a detail from Fig. 12, corresponding to a further step of the method according to the present invention;
Fig. 14 illustrates a further step of the method according to the present invention carried out on the portion of Fig. 7;
Fig. 15 illustrates the partially sectioned perspective view of Fig. 7 at the end of a second sequence of steps of the method according to the present invention;
Fig. 16 illustrates the partially sectioned perspective view of Fig. 8 according to a possible variation of the method according to the present invention;
Fig. 17 illustrates a perspective view of a portion of an anastomotic device applied in a further step of the method according to the present invention;
Fig. 18 illustrates a further step of the method according to the present invention carried out on the portion of Fig. 16;
Figs. 19 and 20 illustrate perspective and enlarged views of a detail from Fig. 18 respectively corresponding to further steps of the method according to the present invention;
Fig. 21 illustrates a further step of the method according to the present invention carried out on the portion of Fig. 15;
Fig. 22 illustrates a perspective view of a portion of an anastomotic device and of a positioning device applied in a further step of the method according to the present invention;
Fig. 23 illustrates a perspective view of the inside of the stomach during a further step of the method according to the present invention;
Fig. 24 illustrates the view from Fig. 23 according to a different point of view;
Fig. 25 illustrates a perspective view from the inside of the stomach of a further step of the method according to the present invention;
Fig. 26 illustrates the view from Fig. 25 according to a different point of view;
Fig. 27 illustrates a perspective view from the inside of the stomach of a further step of the method according to the present invention;
Fig. 28 illustrates a perspective, enlarged and partially transparent view of a detail of the portion of Fig. 1 at the end of a third sequence of steps of the method according to the present invention;
Fig. 29 illustrates a perspective view of the portion of Fig. 1 at the end of the third sequence of steps of the method according to the present invention;
Fig. 30 illustrates a perspective and exploded view of an anastomotic device suitable for being used in some steps of the method according to the present invention;
Fig. 31 illustrates a perspective and enlarged view of a detail of an insertion device suitable for being used in some steps of the method according to the present invention;

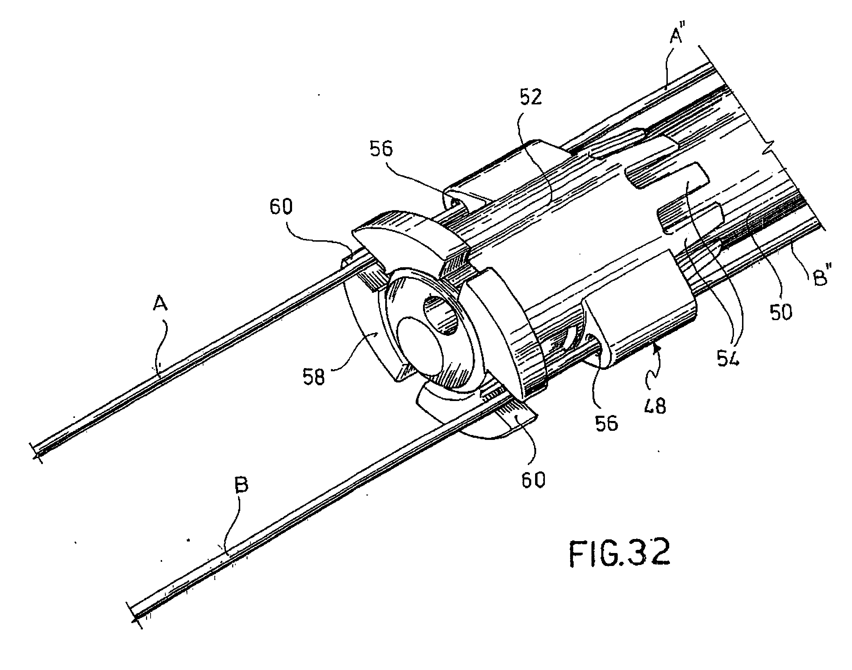

Fig. 32 illustrates a perspective view of a positioning device according to the present invention suitable for being used in some steps of the method according to the present invention;
Fig. 33 illustrates a perspective view of the positioning device of Fig. 32 according to a different point of view and associated to a variant embodiment of a portion of the anastomotic device of Fig. 30;
Fig. 34 illustrates a perspective view of a step of the method according to the present invention, carried out with a variant embodiment of the anastomotic device illustrated in Fig. 30;
Figs. 35 and 36 illustrate two corresponding steps of a method for performing anastomoses in tracts of the digestive tube according to an aspect of the present invention;
Fig. 37 illustrates a possible variant embodiment of an anastomotic device;
Fig. 38 illustrates a possible further variant embodiment of an anastomotic device.

In accordance with a possible aspect of the present invention, an equipment to approximate tissue portions, which are intended to form an anastomosis comprises a guide means suitable for passing through a first tissue portion 12 and a second tissue portion 14 to be connected by anastomosis and an anastomotic device 10 to approximate the first tissue portion 12 and the second tissue portion 14 to be connected by anastomosis. Advantageously, the guide means consists of at least two guide wires A, B suitable for passing through the tissue portions to be connected, for receiving and dragging at least said anastomotic device 10 in order to approximate the first tissue portion 12 and the second tissue portion 14 to be connected by anastomosis.

The equipment according to the present invention is particularly suitable for being used in a method for performing anastomoses in tracts of the digestive tube performed preferably endoluminally, even if a partially laparoscopic, completely laparoscopic or a conventional surgical approach is possible.

The guide wires A, B are located side by side, essentially parallel, and are suitable for being conformed in an open ring shape, also called a loop, which passes through the tissue portions to be connected by anastomosis and which extends between proximal end portions A', B' and distal end portions A", B".

In accordance with what has been illustrated, the guide wires are spaced apart one from the other both in the introducing step and in the passing-through step of the first tissue portion to be connected by anastomosis. According to an alternative embodiment, the guide wires are drawn together and placed side by side to each other also in the introducing and passing-through step of the first tissue portion. In the latter case they can also be connected at the distal end to be subsequently separated when completing the insertion.

According to a possible aspect, the present invention relates to separation and identification means of the proximal end portions A', B' and of the distal end portions A", B" of the guide wires A, B, optionally being part of the aforesaid equipment. Preferably, these separation and identificative means are carried out by means of a sheath suitable for being fitted on the respective end portions.

In particular, the separation and identificative means comprise a first sheath 74 suitable for being fitted on the proximal end portions A', B' of the guide wires A, B. Preferably, the first sheath 74 has a characterizing feature, for example it is made in a certain colour. Furthermore, the separation and identificative means comprise a second sheath 84 suitable for being fitted on the distal end portions A", B" of the guide wires A, B. Preferably, the second sheath 84 has a characterizing feature, for example it is made in a certain colour.

When both the first and the second sheath are provided, the second sheath 84 advantageously has a characterizing feature different from that of the first sheath 74.

In accordance with a possible aspect, the present invention further relates to an insertion device 62 to deploy at least two guide wires A, B, optionally carried out as a component of the aforesaid equipment. The aforesaid device is particularly suitable for being used in a method for performing anastomoses in tracts of the digestive tube.

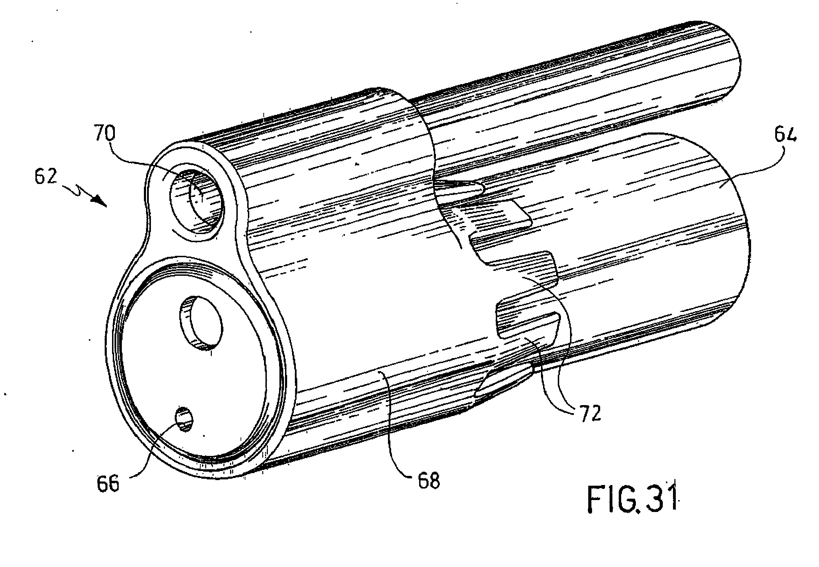

In accordance with a preferred embodiment example, illustrated in Fig. 31, the insertion device 62 comprises an elongated structure preferably made by means of a visualization device 64, for example a gastroscope, provided with a first operative channel 66 suitable for receiving and inserting one of said guide wires. Advantageously, a connecting head 68 suitable for being mounted to a distal end of the elongated structure (visualization device 64) can be further provided. The connecting head 68 is integral with at least one second operative channel 70 suitable for receiving and inserting another guide wire of said guide wires. The operative channel 70 extends essentially along the whole length of the elongated structure to keep the two guide wires distinct during the insertion. Advantageously, the second operative channel 70 is located externally to the elongated structure, made integral with the latter by means of the connecting head 68.

In particular, the first and the second operative channels are used to deploy respectively a guide wire in a method for performing anastomoses in tracts of the digestive tube.

Advantageously, the operative channels are located at a certain distance one to the other, so as to keep the guide wires spaced apart during the insertion and the passing-through of the first tissue portion. Preferably, the second operative channel 70 of the insertion device 62 is placed opposite the first operative channel 66 with respect to the visualization device 64.

In accordance with a possible embodiment, the connecting head 68 of the insertion device 62 is suitable for interference fitting on a distal end of the elongated structure (visualization device 64). In particular, the connecting head 68 comprises elastic tabs 72 which extend from a proximal end of the same connecting head.

In accordance with an alternative embodiment, the insertion device has a single operative channel to insert the guide wires simultaneously and placed side by side. In this case, the guide wires pass through the first tissue portion at a same opening. Optionally, the two guide wires can be initially connected one to the other at the distal end and, subsequently, can be separated at the end of their insertion.

In accordance with a possible embodiment, the equipment according to the present invention comprises an anastomotic device 10, in which an abutment portion 18 is suitable for the abutment against a surface of the first tissue portion 12 and a locking portion 20 is suitable for being placed opposite the abutment portion 18 with respect to the first and second tissue portions 12, 14. The locking portion 20 and the abutment portion 18 are mutually connectable across the approximated first and second tissue portions to keep them connected. At least one of the abutment portion 18 and the locking portion 20 is suitable for being connected to the guide wires A, B to reach the first or the second tissue portion 12, 14.

More particularly, the locking portion 20 and the abutment portion 18 are suitable for being mutually locked in at least two locking positions corresponding to different compression degrees of the first and the second tissue portions.

In accordance with a possible embodiment, the abutment portion 18 of the anastomotic device 10 is suitable for being locked in a direction on said guide wires A, B by which it is dragged until reaching the first tissue portion 12. In other terms, the constraint between the abutment portion 18 and the guide wires allows to drag it to the first tissue portion and to use it to draw the two tissue portions together, allowing the guide wires to be withdrawn in the opposite direction. Furthermore, the locking portion 20 is suitable for sliding along said guide wires A, B and has an interaction portion with a positioning device 48 which pushes it along them.

In accordance with a possible embodiment, the abutment portion 18 is suitable for being locked on the guide wires A, B, for example by means of locking members 61 made respectively integral to a guide wire and suitable for defining an obstacle for the sliding in a direction of the abutment portion 18 on the guide wires.

In accordance with an advantageous embodiment, the abutment portion 18 comprises at least two channels suitable for receiving a guide wire, respectively. Preferably, the channels are located on opposite sides of the anastomotic device. Furthermore, it is advantageously provided that the channels pass through the abutment portion 18.

Preferably, at least one of the guide wires A, B passes through the abutment portion 18 and the locking portion 20 at connecting members between the two portions. In particular, at least one pin 22 extends from the abutment portion 18 and it is suitable for passing through the first tissue portion 12 and the second tissue portion 14 to connect with the locking portion 20 by being inserted in a respective housing 24 of the locking portion. This pin defines a channel 34 therein, which is open on both sides and preferably suitable for receiving a guide wire A, B. The respective housing 24 of the locking portion 20 is suitable for housing said guide wire. According to the illustrated embodiment, the abutment portion 18 and the locking portion 20 are connectable by means of two pins 22 of the abutment portion 18 suitable for inserting in respective housings 24 of the locking portion 20. Each pin 22 defines a channel 34 to receive a guide wire which passes also through the respective housing 24 of the locking portion. Preferably, the two pins are located at two opposite areas or sides of the abutment portion 18 and, similarly, the two housings suitable for receiving the two pins are located at two opposite areas or sides of the locking portion 20.

Preferably, the pin is suitable for snap fitting within the housing 24 of the locking portion 20. The snap fit is for example performed by means of a pin 22 provided with at least one shaped portion 26 suitable for defining a snap fit with elastic tabs 28 associated to the respective housing 24 of the locking portion 20.

Preferably, the elastic tabs 28 extend along the direction of the respective pin 22 away from the abutment portion 18 to extend the housing 24.

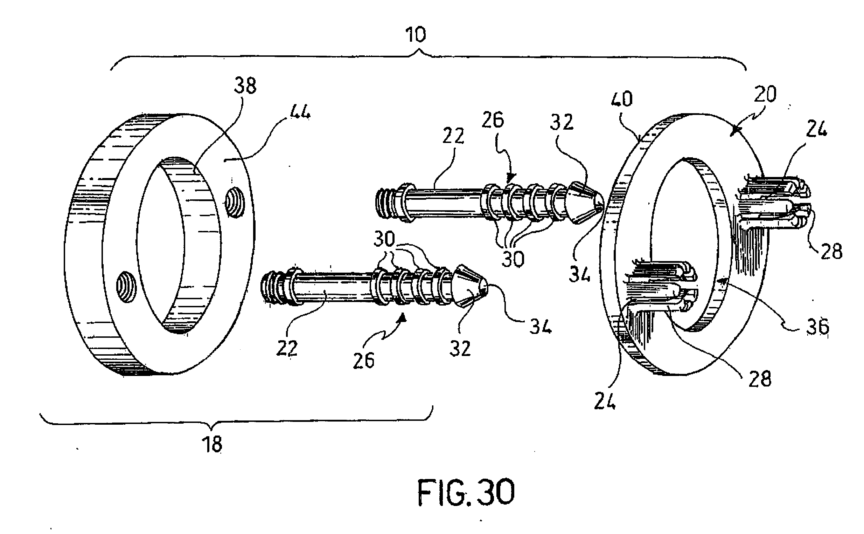

In the preferred embodiment illustrated in Fig. 30, the shaped portion 26 is made by means of a sequence of ring ribs 30. Furthermore, a free end of the pin 22 is shaped so as to promote the snap fitting, for example providing an outwardly tapering or frusto-conical end 32. Furthermore, the elastic tabs 28 are distributed along a peripheral edge of the respective housing 24 and extend in the direction and sense of insertion of the pin 22.

In accordance with a preferred embodiment, the pin 22 is movably mounted, preferably is screwed, on the abutment portion 18.

In accordance with a possible embodiment, the locking portion 20 has an opening 36 to access the area intended for the formation of the anastomosis. Advantageously, the locking portion 20 is essentially ring-shaped. Furthermore, the abutment portion 18 has an opening 38 to access the area intended for the formation of the anastomosis and corresponding to the opening 36 of the locking portion 20. Also the abutment portion 18 is advantageously essentially ring-shaped. In the case where two housings 24 are provided, these are located at opposite parts or sides of the opening 36. In the case where two pins 22 are provided, these are preferably located at opposite parts or sides of the opening 36.

In accordance with a different embodiment, the anastomotic device 10 is a device suitable for approximating the first tissue portion 12 and the second tissue portion 14 and comprises an abutment portion 18 suitable for the abutment against a surface of the first tissue portion 12 to be drawn together. The abutment portion 18 is suitable for being connected to the guide wires A, B to reach the first tissue portion 12. The abutment portion 18 is preferably suitable for being locked on the guide wires A, B by which it is dragged until reaching the first tissue portion 12.

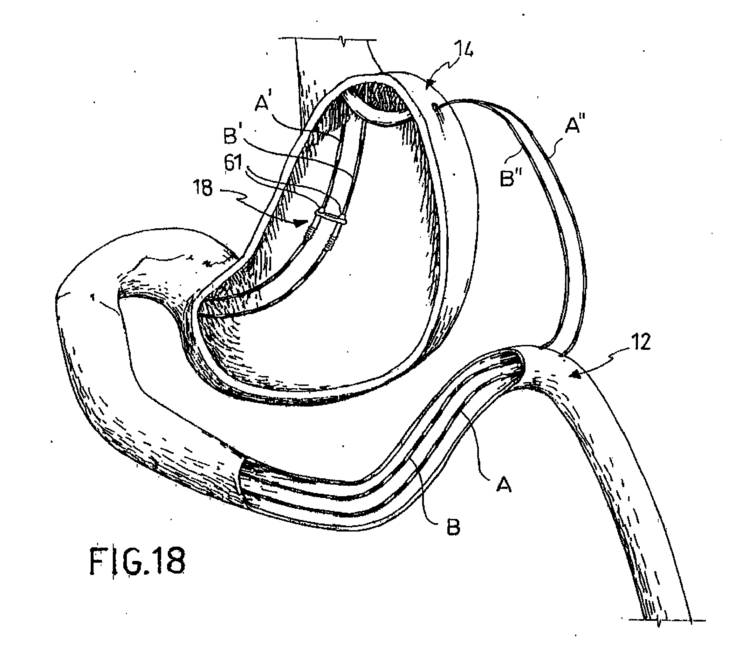

An example of an anastomotic device comprising the abutment portion 18 and the locking portion 20 has been illustrated with reference to Fig. 30. Figs. 18-28, 33, and 34 illustrate possible applications of the anastomotic device 10 or of its embodiments. The anastomotic device 10 is suitable for being used in a method, preferably endoluminally, for carrying out anastomoses in tracts of the digestive apparatus. Optionally, its use is nonetheless possible in partially or wholly laparoscopic techniques or conventional surgical techniques.

Fig. 30 illustrates an exploded view of a preferred embodiment of the anastomotic device 10 in which the abutment portion 18 and the locking portion 20 have been illustrated. Referring in particular to the application of the anastomotic device 10 in a method for performing of a gastrojejunostomy (G-J), or similar methodologies, the abutment portion 18 can also be defined as the intestinal portion, while the locking portion 20 can also be defined as the gastric portion.

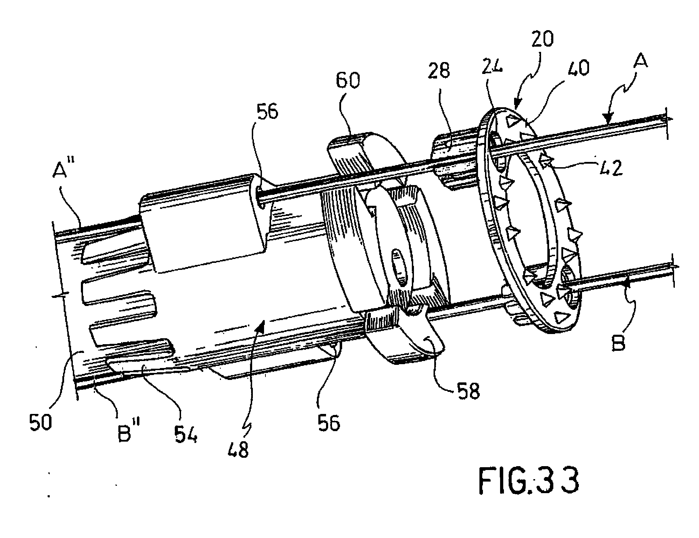

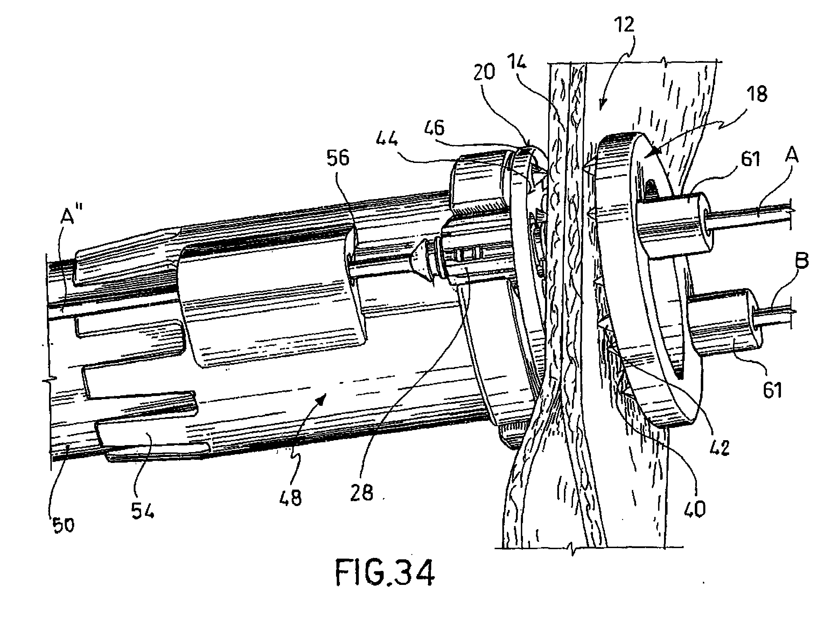

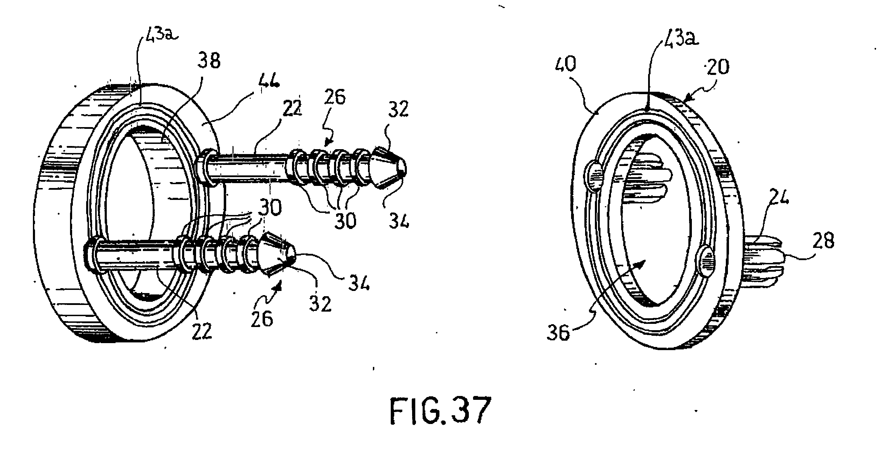

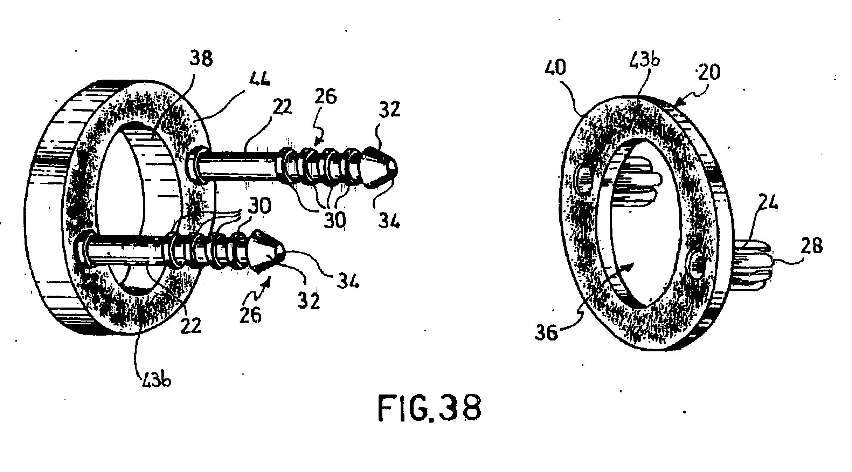

A contact surface of the locking portion 20 suitable for the abutment against a surface of the second tissue portion 14 to be drawn together has been indicated by 40. This contact surface is a surface opposite to the deployment of the elastic tabs 28, where present. In accordance with a preferred embodiment, the contact surface 40 is rough or has pointed members 42 (Figs. 33 and 34). Optionally, annular grooves 43a can be provided (Fig. 37) or, the roughness can be obtained through a layer 43b made of material reported for example in granular form (Fig. 38).

Furthermore, a contact surface of the abutment portion 18 suitable for the abutment against a surface of the first tissue portion 12 to be drawn together has been indicated by 44. This contact surface is a surface located on the same side as the pin 22, where provided. In accordance with a preferred embodiment, the contact surface 44 is rough, or it has pointed members 46 (Fig. 34). Optionally, annular grooves 43a can be provided (Fig. 37) or, the roughness can be imparted through a layer 43b made of material reported for example in granular form (Fig. 38).

With reference to the anastomotic device, it is clear that variations and/or additions to what has been described and illustrated above can be provided.

For example, the pins 22 can be made as one piece with the abutment portion 18. Or, the pins can extend from the locking portion 20 and fit in housings of the abutment portion 18.

The anastomotic device 10 can be made in any type of material suitable for the surgical application. In addition to what has been stated above, in particular in the case of an anastomotic device 10 suitable both for drawing together the tissue portions and keeping the tissue portions connected, both the abutment portion and the locking portion can be made in not-bioabsorbable material, for example in plastic or metallic material. In such a case, the anastomotic device disengages and moves naturally away when the tissue to which it is attached necrotizes. According to a different embodiment, the anastomotic device can be made in such a way as to stay in a steady position.

Alternatively, the choice of the material can be directed towards a bioabsorbable or biofragmentable material, thus providing that the anastomotic device is completely absorbed after a fixed time period.

Finally, the anastomotic device 10 can be partially made in bioabsorbable or biofragmentable material. In particular, it is advantageous to provide for the realization of the connecting members between the abutment portion and the locking portion in bioabsorbable or biofragmentable material, in order to allow the anastomotic device to disengage from the site to with it has been applied after a fixed time period and to move naturally away. In the case illustrated herein, in the annexed drawings, it can be advantageously provided that the pins 22 and/or the housings 24 and/or the elastic tabs 28 are made in bioabsorbable or biofragmentable material.

In accordance with a possible embodiment, the equipment according to the present invention can comprise a guide means and an anastomotic device (in particular an abutment portion 18) spaced apart one from the other, suitable for being mutually associated upon their use. Or, a guide means can be provided in which the anastomotic device (and in particular the abutment portion 18) is pre-assembled or integral on the guide means, so that it can be dragged in a direction and be withdrawn in the opposite direction.

According to a further aspect, the present invention relates to a positioning device 48 to deploy at least one portion of an anastomotic device 10 suitable for drawing together and possibly keeping close to each other a first tissue portion 12 and a second tissue portion 14 intended to form an anastomosis. The aforesaid positioning device is particularly suitable for being part of an equipment to approximate tissue portions, which are intended to form an anastomosis.

In accordance with a possible embodiment, the positioning device is suitable for deploying a locking portion 20 of an anastomotic device 10, for example as described above. Furthermore, the positioning device is suitable for exerting on the locking portion 20 a bias to snap fit it to an abutment portion 18 of the anastomotic device 10 placed opposite the first and second tissue portions.

In accordance with a possible embodiment, the positioning device is suitable for sliding along the guide wires A, B and comprises an elongated structure 50. In the illustrated examples, the elongated structure 50 consists in a visualization device, for example of the gastroscopic kind.

According to possible embodiment, particularly advantageous in the case of the visualization device, the positioning device 48 comprises a head 52 suitable for interference fitting on a distal end of the elongated structure 50 (visualization device). According to a possible embodiment, the head 52 comprises elastic tabs 54 which extend from a proximal end of the same head.

In accordance with an embodiment, the head 52 comprises channels 56 suitable for respectively receiving a guide wire.

Furthermore, the head 52 comprises a distal end defining a thrust surface 58 for the locking portion 20. In the case where the locking portion 20 comprises housings 24 to receive guide wires and elastic tabs 28 to snap locking on the abutment portion 18, the distal end of the head 52 comprises at least one opening 60 to receive the elastic tabs of the locking portion. In the example illustrated in the Figures, two housings of the locking portion and two relative openings 60 of said head 52 are provided.

In accordance with possible variations to what has been illustrated, the head 52 can be applied to any kind of elongated structure.

The positioning device allows adapting already existing structures, such as visualization devices (gastroscopes), in particular with the aim to carry out an endoluminal technique to perform anastomoses of tracts of the digestive tube.

The elongated structure can nonetheless be realised both by a flexible structure, and by a rigid structure, in order to exert a higher bias on the anastomotic device, and by a "stiffening" structure, that is a flexible structure suitable for becoming rigid.

The positioning device according to the present invention is easy and efficient and, being able to slide on guide wires, it allows for a proper alignment of the two portions of the anastomotic device.

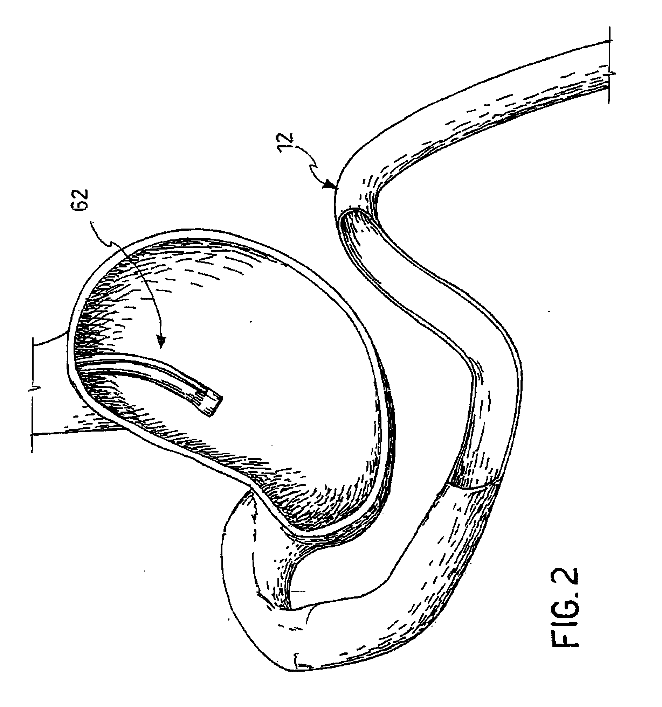

The mode of use of an insertion device according to the preferred embodiment illustrated and previously described is described below. The application example relates to a method for performing anastomoses in tracts of the digestive tube, and in particular to some steps of a method for performing endoluminally a gastrojejunostomy, for example as illustrated in Figs. 2-7.

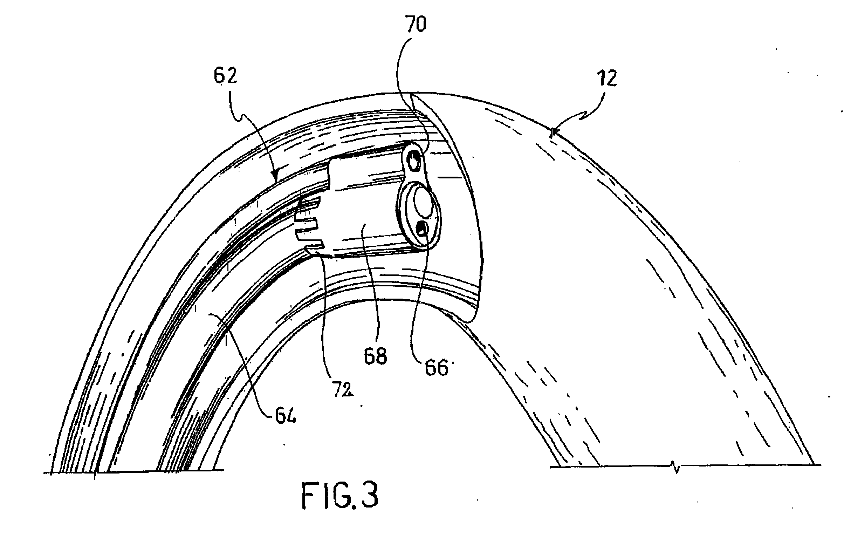

The insertion device 62 is assembled externally to the patient, by inserting the connecting head 68 on the distal end of the elongated structure (visualization device 64 - Fig. 31). Subsequently, the insertion device 62 is introduced in the oesophagus and in the stomach (Fig. 2), continuing to the jejune and to the first tissue portion 12 (Fig. 3).

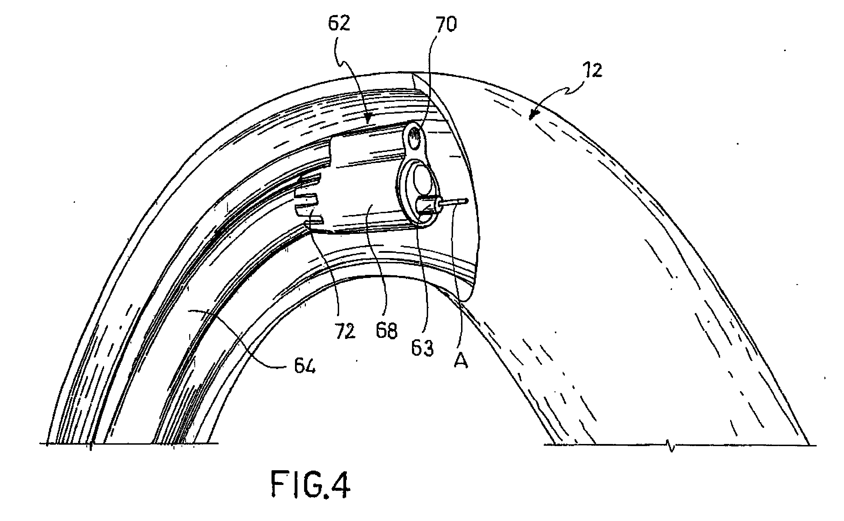

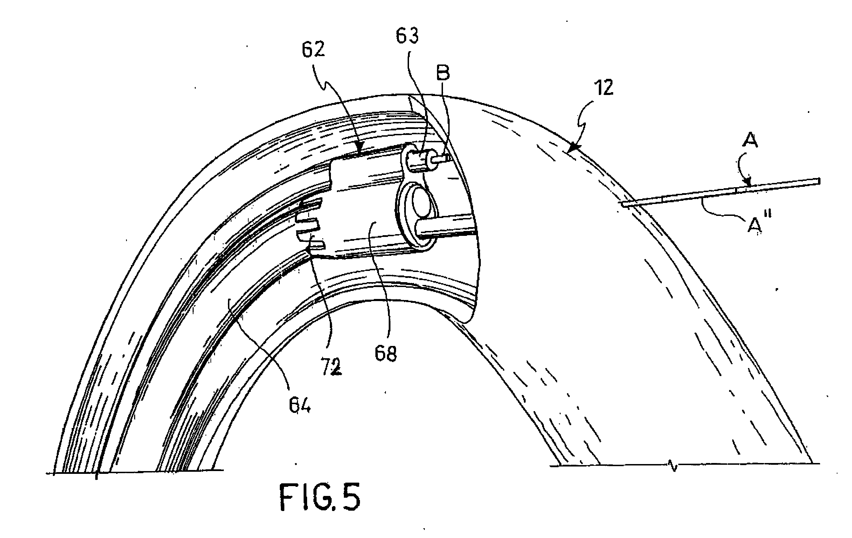

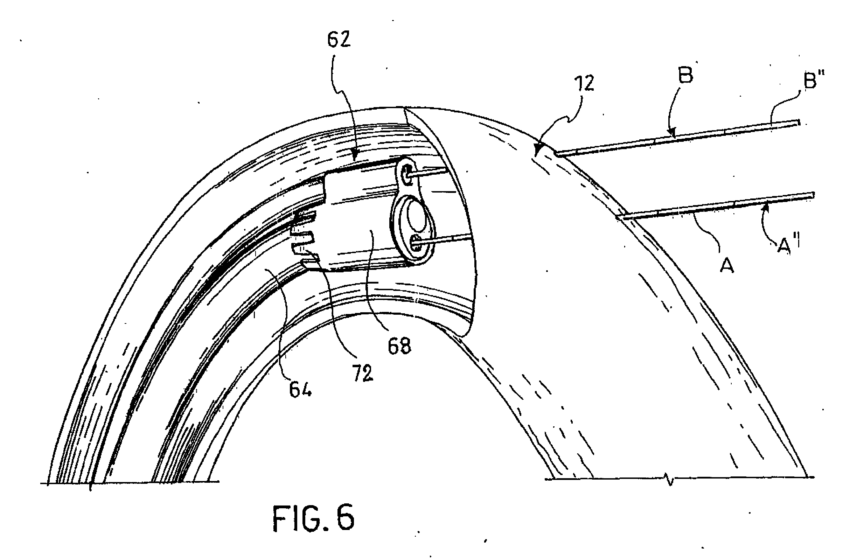

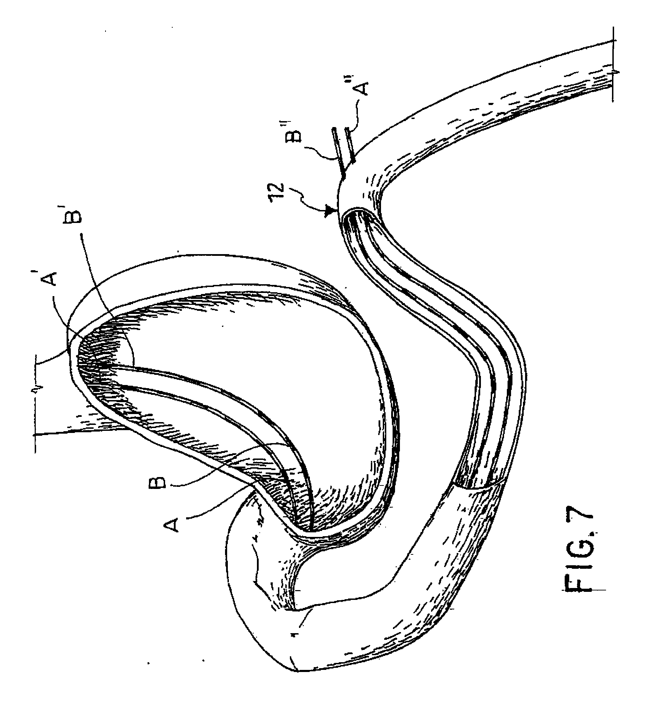

A guide wire A is inserted through the first operative channel 66 until a distal end of the guide wire projects from the distal end of the insertion device 62 (Fig. 4) and passes through the wall of the first tissue portion. A distal end portion A" of the guide wire extends beyond the wall of the first tissue portion. Furthermore, a second guide wire is inserted through the second operative channel 70 until a distal end of the guide wire projects from the distal end of the insertion device 62 (Fig. 5) and passes through the wall of the first tissue portion. A distal end portion B" of the second guide wire extends beyond the wall of the first tissue portion (Fig. 6). The insertion device is then retracted (Fig. 7).

Before introducing the guide wires, the first tissue portion wall is punched, for example by means of radiofrequency or via other devices, optionally introduced by means of the same insertion device 62. For example, a sheath 63 containing a radiofrequency needle is inserted along the first operative channel 66 to the first tissue portion wall. The radiofrequency needle punches the wall, and subsequently a guide wire A is inserted along the sheath until passing through the first tissue portion wall, at the hole punched by the radiofrequency needle. The same procedure can be followed in the guide wire B positioning through the second operative channel 70.

In accordance with a different embodiment and applicative example, the guide wires are inserted drawn together and placed side by side to each other through a single operative channel, for example of an insertion device or a visualization device. Before introducing the guide wires, the first tissue portion wall is punched in a single point, for example by means of radiofrequency or through other devices, optionally introduced by means of the same insertion device.

The insertion device comprising at least two operative channels allows to position two or more guide wires, keeping them as distinct one from the other as possible. Furthermore, it allows carrying out endoluminal techniques to perform anastomoses of tracts of the digestive tube, also using pre-existent tools such as visualization devices (gastroscopes). In virtue of the presence of the connecting head, it is possible to use at least two operative channels which allow to simultaneously position at least two guide wires, achieving a controlled distance, in particular between the points in which the guide wires pass through the tissue portions.

The mode of use of an anastomotic device according to the preferred embodiments illustrated and according to the two above described aspects is described below. It is further described the mode of use of a positioning device as described above. The applicative example refers to a method for performing anastomoses in tracts of the digestive tube, and in particular to a method for performing endoluminally a gastrojejunostomy, for example as illustrated in Figs. 18-28.

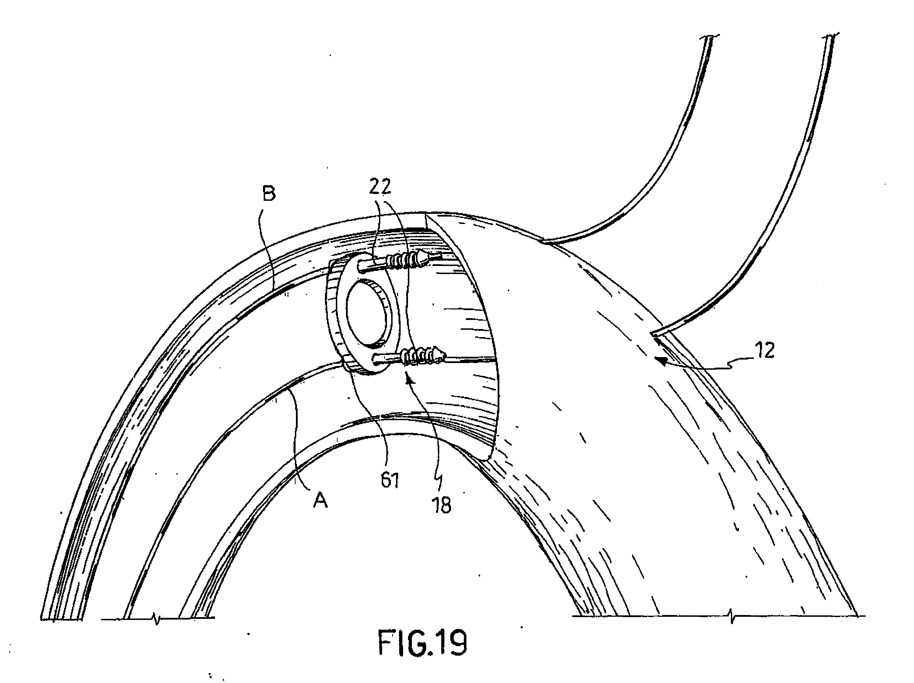

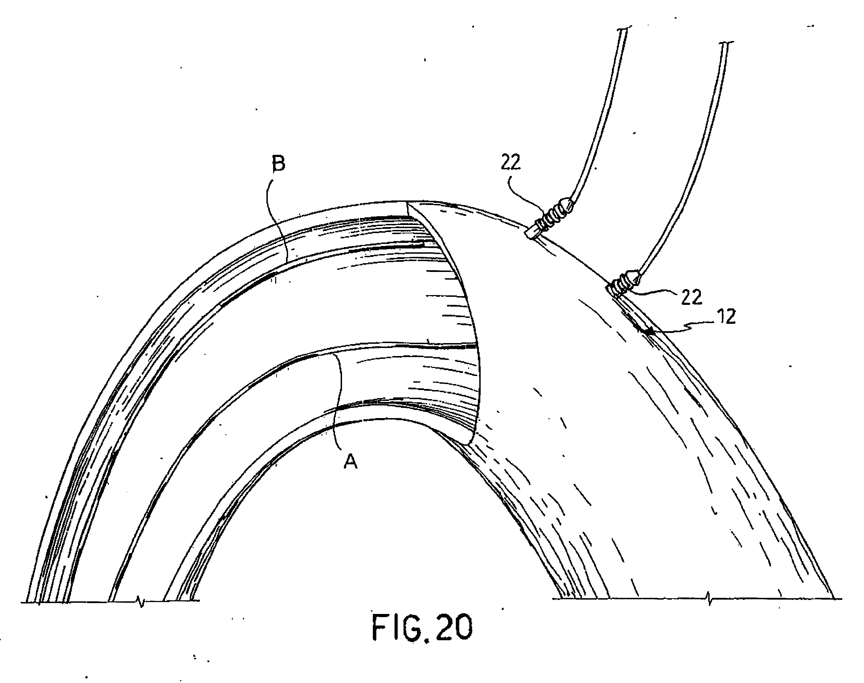

In Fig. 18 a portion of the digestive apparatus, comprising the stomach and a tract of the intestine corresponding to a jejune tract, is illustrated. A guide means, for example comprising two guide wires, has been positioned starting from a natural orifice, such as the oesophagus, and forms a loop which passes through the first tissue portion 12 and the second tissue portion 14. The two guide wires A and B extend between proximal end portions A' and B' and distal end portions A" and B". The abutment portion 18 of the anastomotic device 10 is inserted on proximal end portions of the guide wires and locked in a direction, for example by means of the locking members 61. Alternatively, other locking means can be provided, for example integrated in the anastomotic device structure. Subsequently, the abutment portion 18 is drawn together to the first tissue portion 12 pulling distal end portions of the guide wires (Fig. 19) until the pins 22 insert and pass through the first tissue portion 12 (Fig. 20).

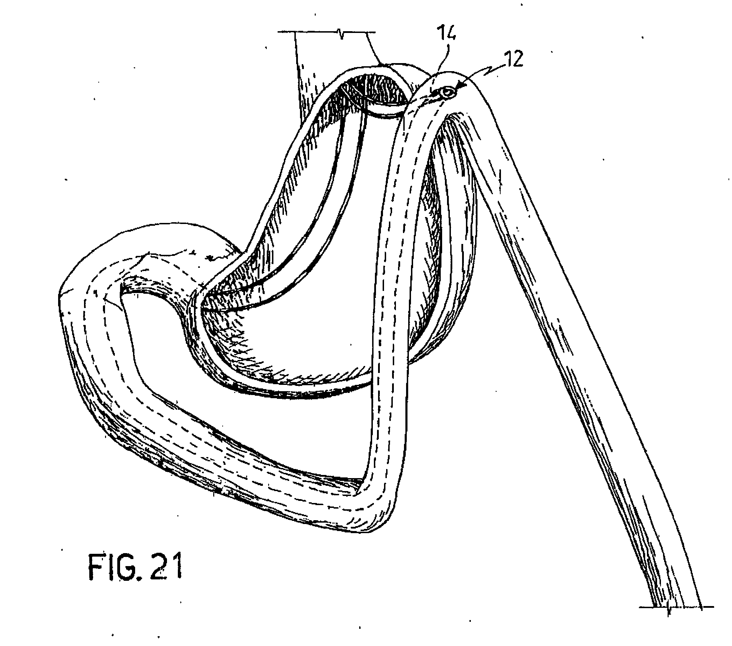

The contact surface 44 abuts internally against the first tissue portion 12, therefore a further traction performed on the distal end portions of the guide wires causes the drawing of the first tissue portion 12, dragged by the abutment portion 18, together with the second tissue portion 14 (Fig. 21). In other terms, Figs. 18-21 illustrate an application of an anastomotic device 10 suitable for drawing together a first tissue portion and a second tissue portion intended to form an anastomosis.

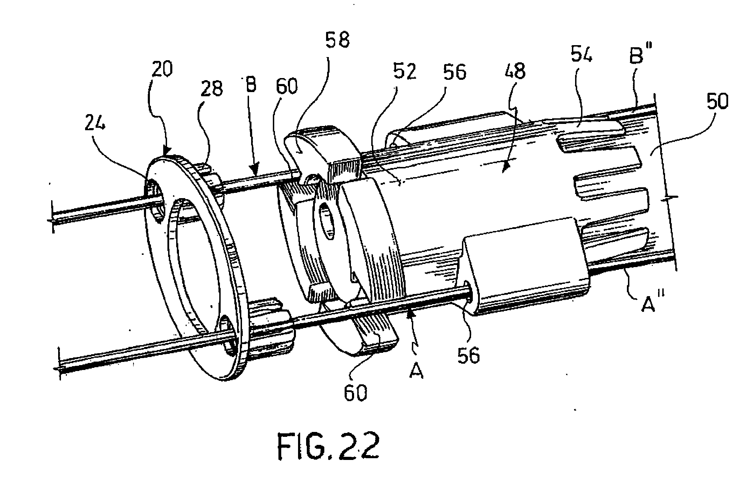

The advantageous conformation of the anastomotic device 10 makes it possible to be used as a device not only to approximate the two tissue portions, but also to keep connected the two tissue portions. This application is further illustrated in Figs. 22-28, in which the application of the locking portion 20 is shown, preferably biased in position along the guide wires by the positioning device 48. The locking portion 20 is inserted on the distal end portions A" and B" of the guide wires by means of the housings 24. Subsequently, also the head 52 is inserted on the distal end portions of the guide wires by means of the channels 56. Finally, the head 52 is fitted on the distal end of the elongated structure 50, or gastroscope (Fig. 22). The positioning device 48 interacts with the locking portion 20 via the thrust surface 58 and the elastic tabs 28 fit in the respective openings 60.

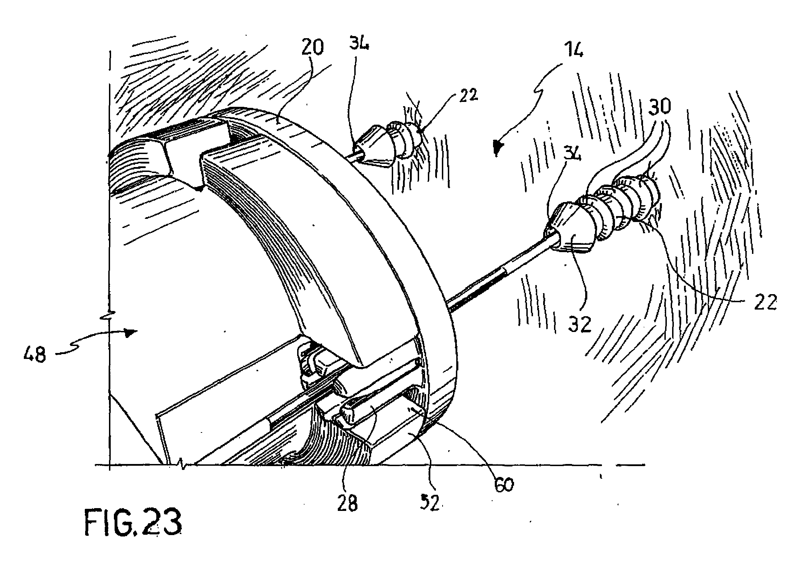

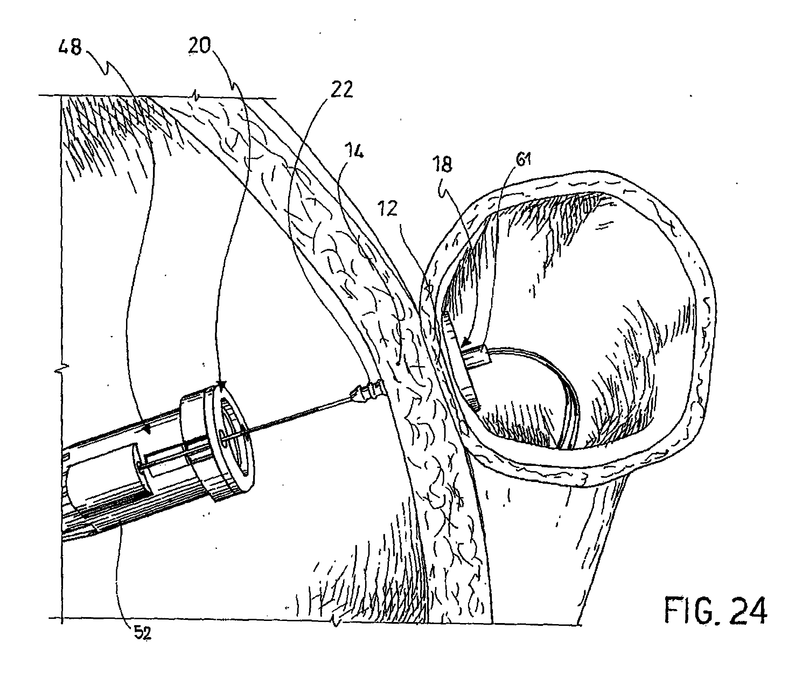

The head 52 biases the locking portion 20 in position by sliding on the guide wires until approaching to the second tissue portion 14, opposite the abutment portion 18, i.e. on the stomach side (Figs. 23 and 24).

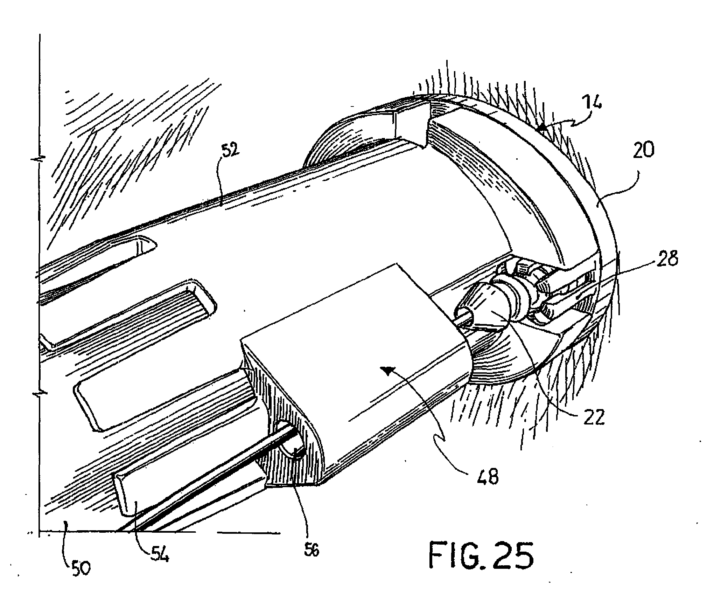

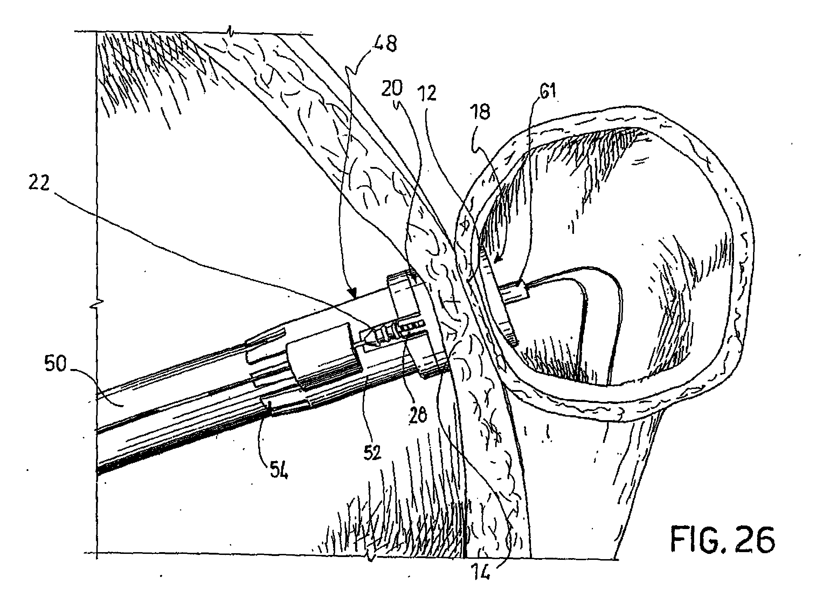

Keeping to bias the elongated structure 50 along the guide wires, the pins 22 fit in the housings 24 of the locking portion and the frusto-conical end 32 enlarges the elastic tabs 28, which abut between a ring rib 30 and the next one, carrying out a snap fit (Figs. 25 and 26). The bias exerted on the locking portion 20 via the head 52 and the elongated structure 50 allows to select one of the mutual possible positions of the abutment portion 18 and the locking portion 20. In other terms, the presence of ring ribs 30 allows to define at least two relative positions between the abutment portion 28 and the locking portion 20, in order to establish an optimum compression degree of the tissues, which can be assessed as a function of their more or less ischemic coloration.

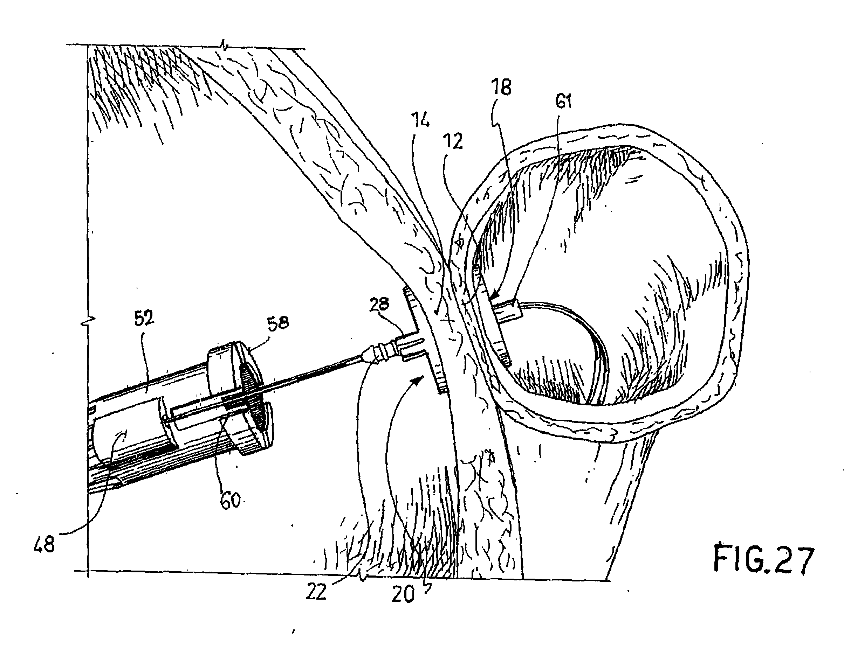

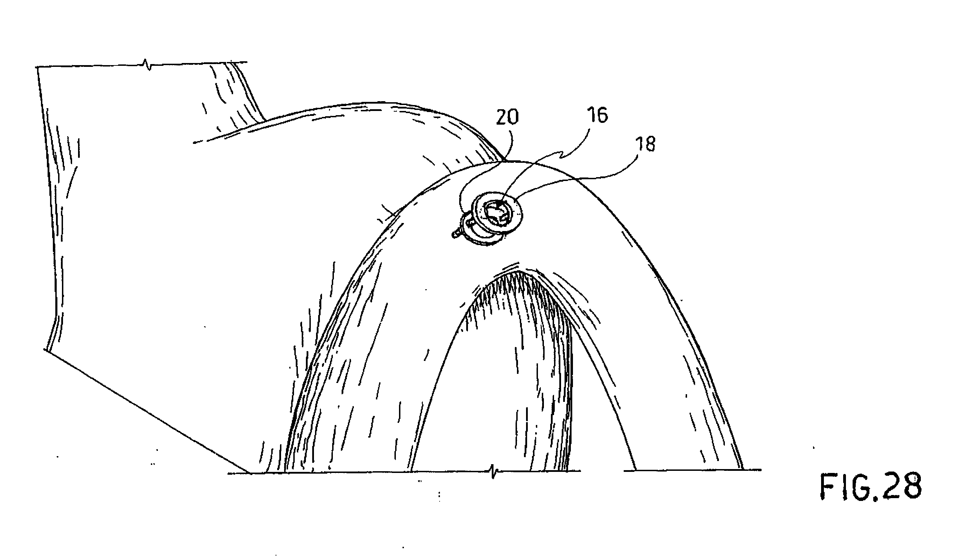

By withdrawing the positioning device 48, the locking portion 20 continue to be attached to the abutment portion 18 allowing to keep connected the two tissue portions (Figs. 27 and 28). Subsequently, the anastomosis can be completed as it will be described below.

The advantageous provision of an anastomotic device according to the above described aspects allows to provide an efficient and easily usable tool, and moreover a tool suitable for performing endoluminally anastomoses of tracts of the digestive tube, even if its use is possible also in other techniques, for example partially or wholly laparoscopic techniques or conventional surgical techniques.

A first object of the device is to provide an abutment portion which allows drawing together two tissue portions to be connected by anastomosis. According to this aspect, the advantageous provision that it may be connected, or even locked, on at least two guide wires, allows to control and direct the anastomotic device throughout the path within the apparatus, both to approximate the two tissue portions and to connect the two tissue portions.

Providing an annular conformation which connects on two sides opposite two guide wires, it is possible to perform the anastomosis within the anastomotic device used in order to draw the tissues together. A possible performing step of the anastomosis will be described below in the detail of the description of the method according to the present invention.

A further advantage is given by the presence of two portions suitable for being connected or coupled one to the other in such a way that the same device can be used both to approximate the two tissue portions and to keep the two tissue portions drawn together. The locking is easy and effective, because it is done in a snap manner, furthermore with the possibility to determine different compression degrees of the tissues.

The latter aspect may be independent from the presence of one or more guide wires, even if the combination of the two aspects allows to accurately control and direct also the locking step, in particular in the case where the guide wires pass through the pins and the connecting housings between le two portions. As a consequence, it is further achieved a very compact anastomotic device.

According to a further aspect, the present invention further relates to a method for performing anastomoses in tracts of the digestive tube. A first step of the aforesaid method provides for the introduction, through a natural orifice or other luminal structures, at least two guide wires A and B essentially parallel. The guide wires can be at least partially placed side by side to each other at a certain distance one from the other, or placed side by side to each other and drawn together. The guide wires extend between proximal end portions A' and B' and distal end portions A" and B", and pass through a first tissue portion 12 and a second tissue portion 14 in which to perform the anastomosis.

The aforesaid method provides the insertion of an anastomotic device along the two guide wires and dragging it to the anastomotic site, drawing the first tissue portion and the second tissue portion together and performing an anastomosis. In the case where the anastomotic device, and in particular the abutment portion 18, is re-assembled on the guide wires, it is sufficient to drag it, drawing the first tissue portion and the second tissue portion together.

In particular, the two guide wires are located so as to form a loop with the end portions at natural orifices, or other orifices. The loop passes through the first tissue portion 12 and the second tissue portion 14 to be connected. The anastomotic device is inserted along the two wires to perform preferably endoluminally the anastomosis.

The distal ends of the two guide wires are first inserted to the first tissue portion 12 and introduced therethrough until passing it through and projecting oppositely.

Preferably, the two guide wires are inserted through an insertion device 62, as described previously, at least at least until reaching and passing through the first tissue portion 12. The guide wires are inserted through the operative channels of the insertion device and pass through the first tissue portion wall 12 previously punched, in such a manner that a distal end portion A" and B" of the guide wires passes through the first tissue portion. The punching of the tissue wall can occur by means of radiofrequency needles inserted in the same operative channels in which the guide wires are subsequently inserted. After that the guide wires have passed through the first tissue portion wall, the insertion device is then retracted. In this case, to each guide wire corresponds an opening through the first tissue portion, and the guide wires are at least partially mutually spaced apart.

Optionally, the guide wires can be introduced by means of a same operative channel, placed side by side to each other and drawn together, so as to pass through the first tissue portion at a common opening. The distal end portions of the guide wires can be connected, at least during the introduction step.

According to a possible embodiment, preferably before passing through the second tissue portion, a separation and identificative step of the proximal end portions A' and B' of the guide wires which come out from the luminal structure is provided. Preferably this step is performed introducing the first sheath 74 on the guide wires starting from a proximal end of the same guide wires. The first sheath has a characterizing element, for example it is made in a prefixed colour.

To complete the loop, the insertion of a grasping device 76 through the second tissue portion 14 to be connected to grasp the distal end portions A" and B" of the two guide wires is advantageously provided.. In order to make the grasping device pass through the second tissue portion, the creation of a hole 78 in the second tissue portion 14, and optionally its enlargement by means of a balloon catheter 80, preferably introduced through a operative channel of the grasping device, is advantageously provided.

According to a possible embodiment, the grasping device is a visualization device, for example of the gastroscopic kind, in which an operative channel is used to insert the balloon catheter 80 to enlarge the hole, and subsequently a snare 82 for grasping the distal end portions A" and B" of the guide wires. When the loop closes on the distal end portions of the guide wires, the loop, together with the grasping device, is retracted through the hole 78 to the orifice through which it had been introduced. The guide wires are then located to form a loop with distal and proximal end portions preferably brought back in the same orifice. Optionally it is possible to avoid enlarging the hole 78 making only the snare 82 pass through it. The distal end portions of the guide wires are then introduced into the snare by a laparoscopic approach, and subsequently the snare is reclosed and brought back, completing the loop.

According to a possible embodiment, a separation and identificative step of the distal end portions of the guide wires is provided which, after passing through the first and the second tissue portions, come out from the luminal structure. Advantageously, this separation and identificative step of the distal end portions of the guide wires is performed introducing the second sheath 84 on the guide wires starting from the distal end of the same guide wires. The second sheath has a characterizing feature, for example a colour different from that of the first sheath 74.

Once the loop has been formed, the anastomotic device is inserted on the two guide wires so as to draw and optionally keep drawn the first tissue portion and the second tissue portion together. According to an advantageous embodiment, the anastomotic device used is an anastomotic device 10 according to what has been previously described. For example, a first portion of the anastomotic device, or abutment portion 18, is inserted and locked in a direction on the proximal end portions A' and B' of the guide wires and dragged by pulling one of the free ends of the loop, in particular pulling the distal end portions A" and B". In the case where the first sheath 74 is provided, the abutment portion of the anastomotic device is inserted on the proximal end portions of the guide wires after removing the first sheath 74. Optionally, the abutment portions 18 are pre-associated to the guide wires in a way so as that, by dragging the distal ends of the guide wires, the abutment portion is dragged and drawn together to the tissue portions to be connected.

In the case where the distal end portions of the guide wires are connected, it is possible to divide them after forming the loop, or subsequently to the formation of the same.

The abutment portion of the anastomotic device is dragged until abutting and partially inserting into the first portion of the tissue to be connected (in the case of an anastomotic device as described above, the pins 22 insert through the punchings previously carried out, through which also the guide wires pass). Subsequently, the abutment portion of the anastomotic device is further dragged, together with the first tissue portion to be connected, until being drawn together to the second tissue portion to be connected.

In the case where a first sheath 74 is provided, the latter is preferably repositioned on the proximal end portions of the guide wires when the abutment portion of the anastomotic device has drawn the two tissue portions to be connected together.

According to an embodiment in which the use of the anastomotic device is provided as described above, a second portion of the anastomotic device, or locking portion 20, is inserted on the distal end portions of the guide wires and biased towards the abutment portion of the anastomotic device. In the case where a second sheath 84 is provided, the locking portion of the anastomotic device is inserted on the distal end portions A" and B" of the guide wires after removing the second sheath 84.

According to a possible embodiment, the locking portion of the anastomotic device is snap locked on the abutment portion of the anastomotic device, preferably in one of the possible mutual positions, selecting the compression degree of the first and the second tissue portions.

According to a possible embodiment, the locking portion of the anastomotic device is biased by a positioning device, as described above.

Advantageously, the abutment portion and the locking portion are essentially ring-shaped, therefore the anastomosis is formed at respective openings of the two portions, as it will be described below.

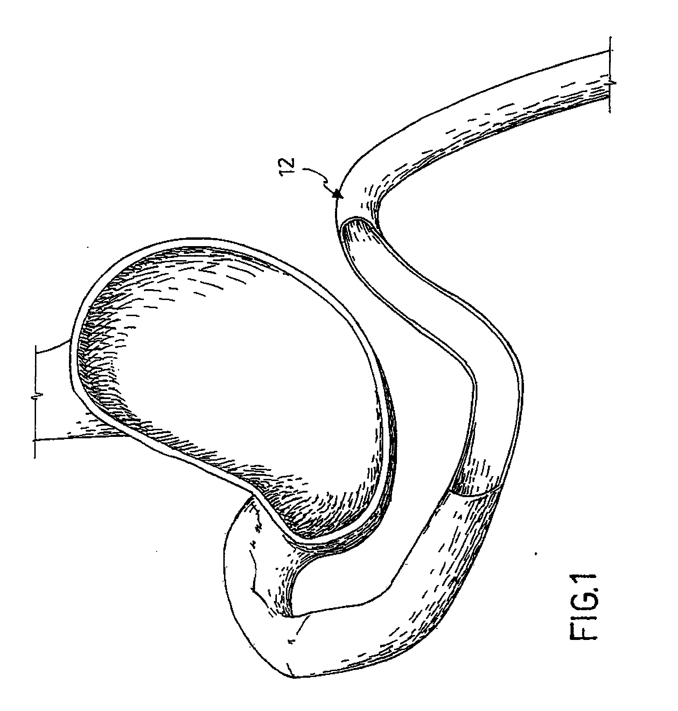

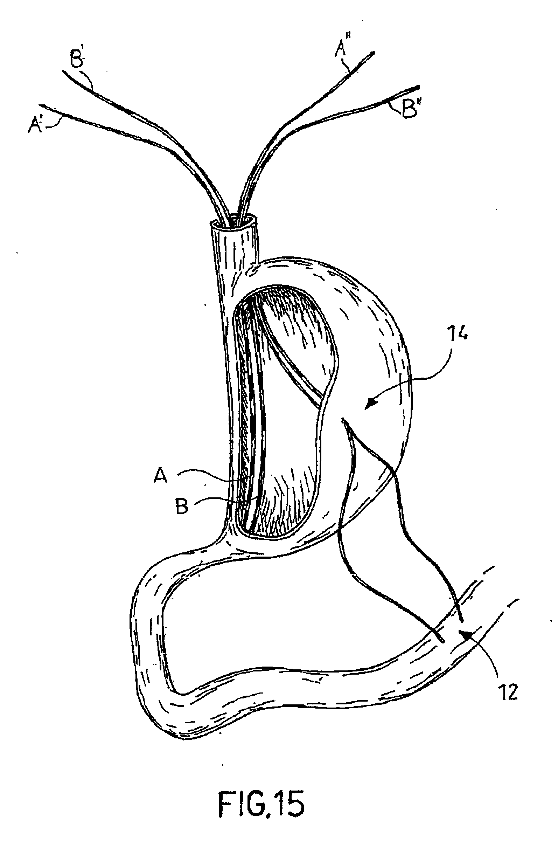

With reference to the annexed Figs., the aforesaid method can be used for example in a performing step of a gastro-jejuno-anastomosis (G-J), advantageously endoluminally. In fact, Fig. 1 illustrates a portion of the digestive apparatus comprising the oesophagus, the stomach and a tract of the intestine corresponding to the jejune. The two guide wires A and B are introduced through the oesophagus, which represents a natural orifice, or through another orifice, also artificial, until reaching the stomach and a tract of the jejune (Fig. 7). Subsequently, the two guide wires A and B are loop-shaped with distal A", B" and proximal end portions A', B' debouching through the aforesaid orifice (Fig. 15). The loop passes through the portions to be connected, respectively a jejunostomy and a gastrostomy.

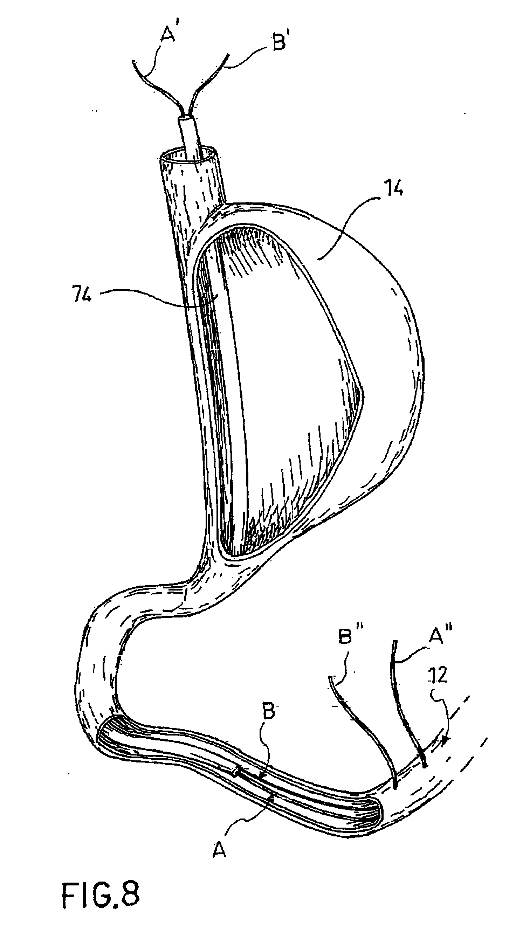

In particular, the two guide wires A and B are introduced through the oesophagus, the stomach and a tract of the jejune and one their distal end is made to pass through the jejune wall corresponding to the first tissue portion to be connected (Fig. 6), for example after punching this wall through radiofrequency needles as described above. This step is performed preferably via an insertion device 62 as illustrated in Figs. 2-6, previously described, or keeping the guide wires drawn together in a single operative channel. Fig. 7 illustrates the two guide wires after that the insertion device has been retracted. Fig. 8 illustrates a possible next step in which the first sheath 74 is inserted on the proximal end portions of the guide wires.

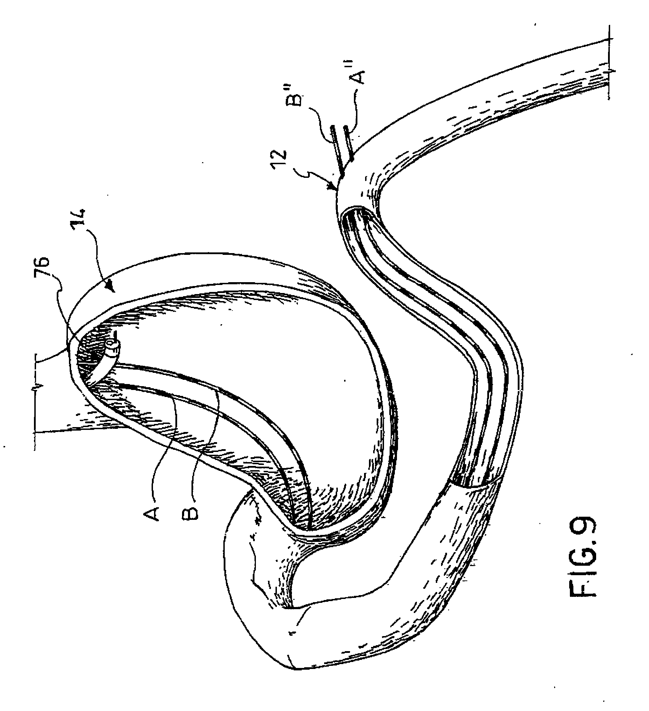

Subsequently, a gastrostomy is created at the second tissue portion 14 to be connected. The gastrostomy can be performed as illustrated in Figs. 9-11, in which a grasping device, for example comprising a gastroscope, is inserted into the stomach and through its operative channel the tool for punching the hole (radiofrequency needle or other) is inserted.

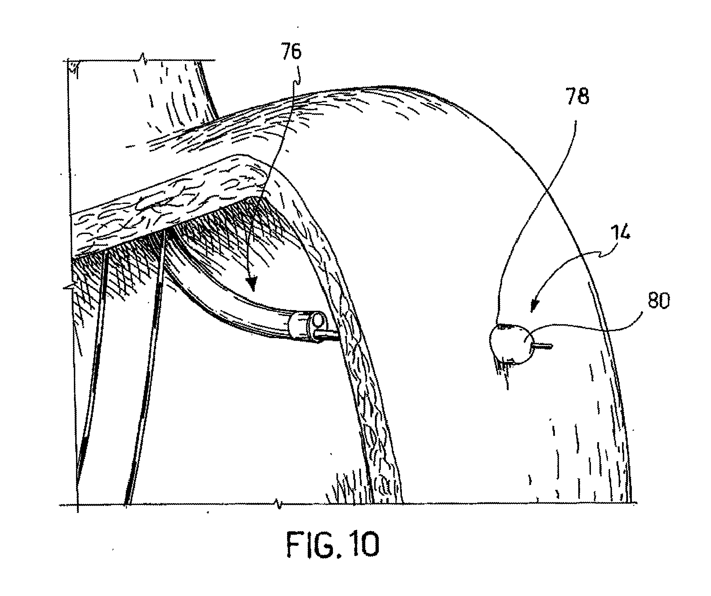

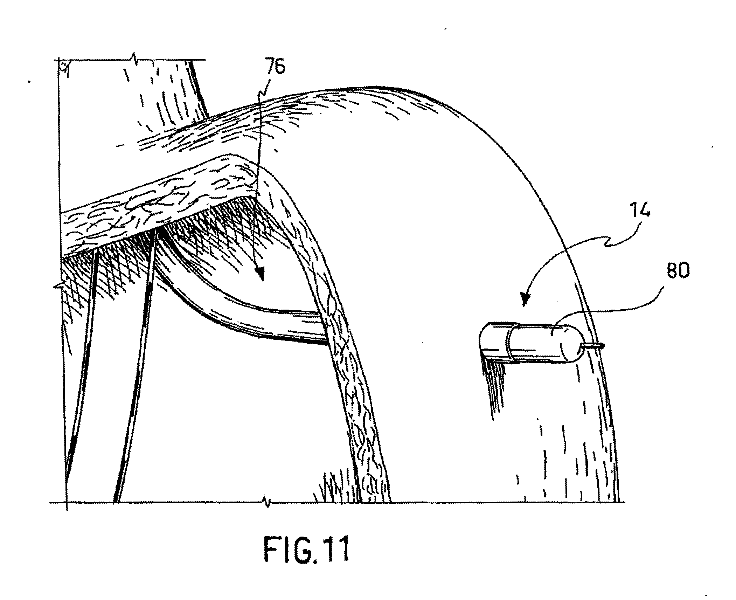

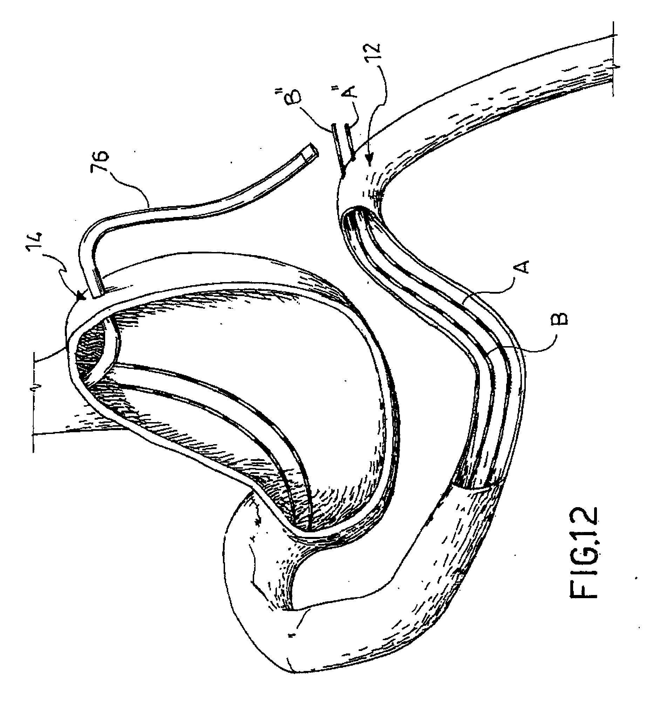

Optionally, the grasping device can provide a balloon catheter 80 which is inflated to enlarge the gastrostomy (Fig. 10) and allow the passing-through of the device (gastroscope - Figs. 11-12) which reaches the end portions of the two guide wires which pass through the first tissue portion.

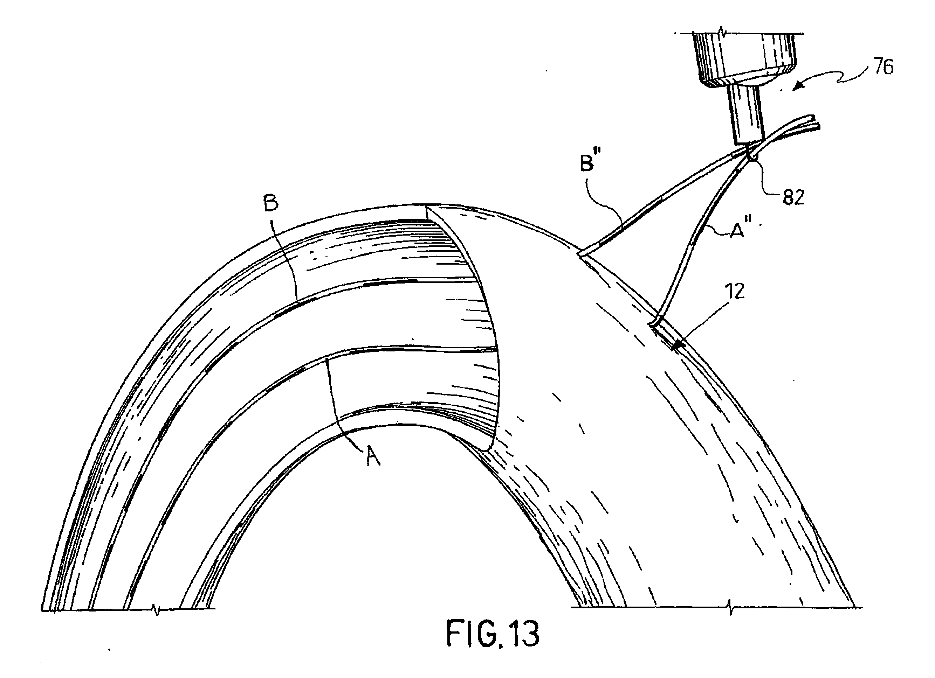

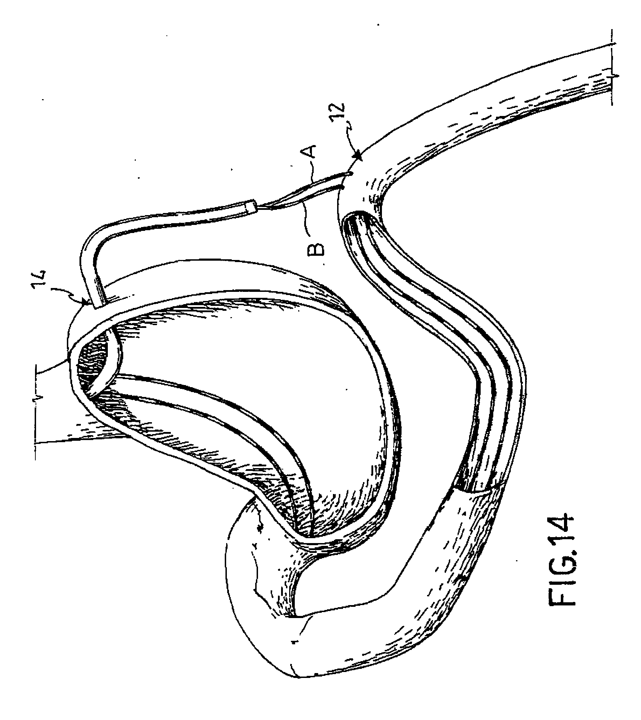

A snare 82 grasps the distal end portions A" and B" of the guide wires (Fig. 13) and drags them back Fig. 14) until passing through the gastrostomy and reconnect in the orifice (oesophagus) in which the proximal end portions of the guide wires are located.

According to an alternative not shown, in which a laparoscopic supervision is provided, the gastrostomy is not enlarged and only the snare is made to pass through it. Subsequently, the surgeon laparoscopically individuates the distal end portions A" and B" and inserts them into the snare, under laparoscopic control. Finally, the snare is closed again and dragged back through the gastrostomy so as to form the loop, as provided in Fig. 15.

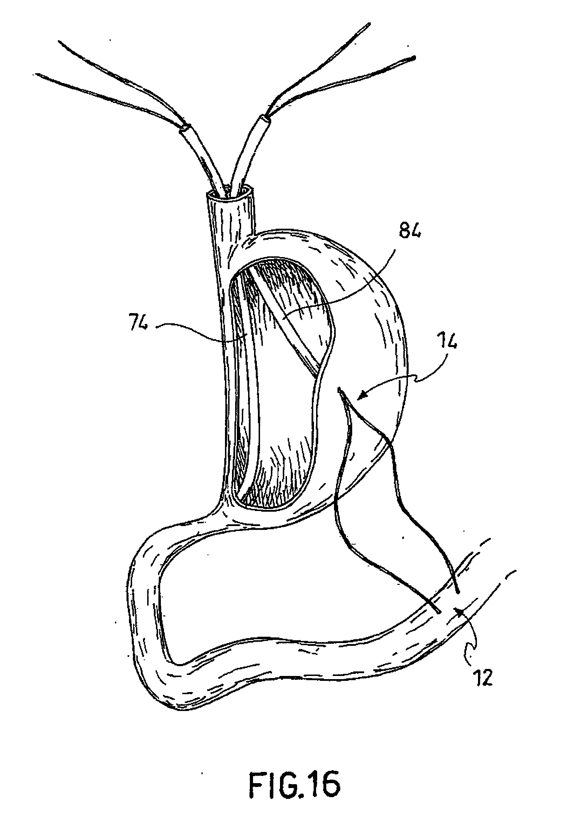

Fig. 15 illustrates the loop formed by the guide wires, while Fig. 16 illustrates a possible step in which the second sheath 84 is inserted on the distal end portions.

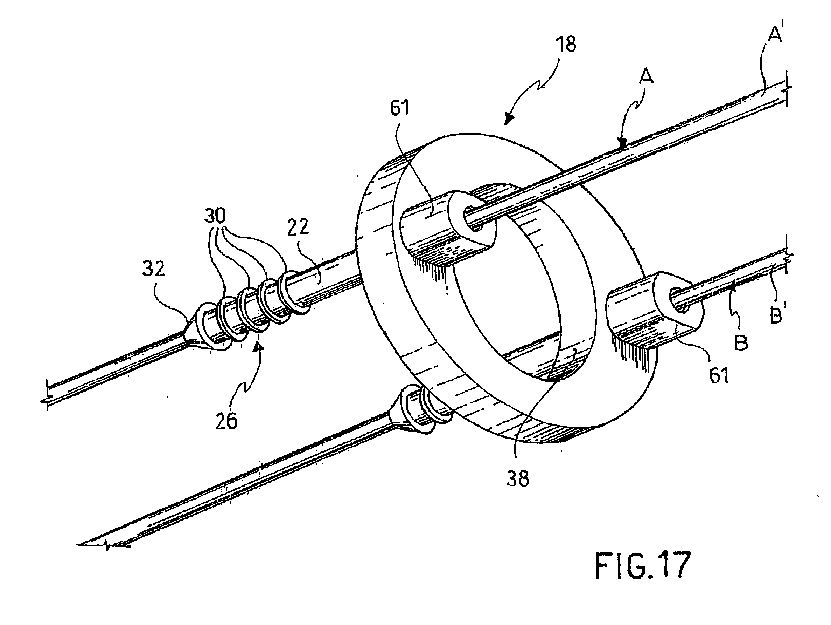

Once the loop has been formed, the two tissue portions are drawn together using the abutment portion 18 of the anastomotic device 10. This abutment portion is inserted on the proximal end portions A' and B' of the guide wires and locked in a direction thereupon, for example by means of locking members 61 (Fig. 17). Or, the abutment portion 18 is pre-assembled on the guide wires. Subsequently, the distal end portions A" and B" of the guide wires are pulled until the abutment portion reaches the jejunostomy and the first tissue portion 12 to be connected (Figs. 18 and 19). The traction on the distal end portions A" and B" of the guide wires continues until the abutment portion 18 abuts against the internal walls at the jejunostomy (Fig. 20).

The distal end portions A" and B" of the guide wires are further pulled until the abutment portion 18 drags the jejune, drawing together the first portion 12 of the jejune and the second portion 14 of the stomach. The abutment portion 18 enters partially the gastrostomy (Fig. 21 - pins 22).

To keep the two tissue portions drawn together and to allow the formation of the anastomosis, the locking portion 20 of the anastomotic device 10 is inserted on the distal end portions A" and B" of the guide wires and biased towards the abutment portion 18 of the anastomotic device 10. Preferably, the positioning device 48 is used as illustrated in Fig. 22. Figs. 23 and 24 illustrate the drawing-together step of the locking portion 20 and Figs. 25 and 26 illustrate the locking step in which the locking portion 20 of the anastomotic device 10 is snap locked on the abutment portion 18 of the anastomotic device 10.

Advantageously, the locking portion 20 is snap locked on the abutment portion 18 in one of the possible mutual positions selecting the compression degree of the first and second tissue portions. In fact, the frusto-conical end 32 enlarges the elastic tabs 28 which insert beyond the first annular rib 30. This position corresponds to the position in which the minimum compression degree of the tissue portions to be connected is exerted. Further biasing the locking portion 20 on the abutment portion 18, the elastic tabs 28 pass beyond a second annular rib 30, abutting beyond it in a second position in which the two tissue portions are lightly more compressed. The presence of further ring ribs allows choosing between more positions relative of the abutment portion and of the locking portion corresponding to increasing compression degrees of the two tissue portions to be connected.

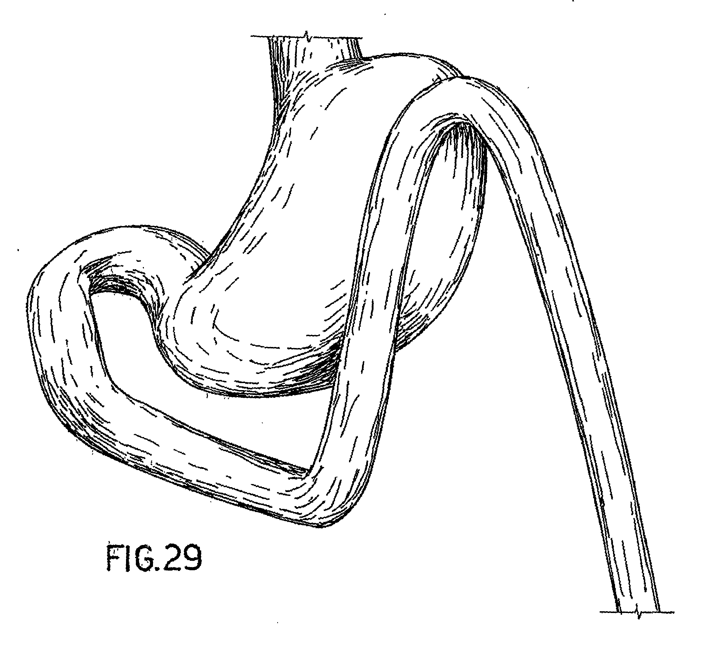

The positioning device 48 is then withdrawn (Fig. 27) and the two portions of the anastomotic device keep the two tissue portions connected (Figs. 28 and 29).

The final step of the method provides the performing of the anastomosis. Using the anastomotic device as described above, the anastomosis is performed at one of the opening of the two annular structures of the anastomotic device.

According to a preferred embodiment, the anastomosis is performed from the side of the jejune, introducing for example a radiofrequency needle suitable for punching the wall. Optionally a balloon catheter is subsequently introduced to enlarge the punching and/or a further tool to remove part of the tissue.

According to an alternative the anastomosis can be performed on the side of the stomach. In this case it is possible to provide the introduction of a shielding device in the jejune, in order to avoid that the punching affects also the jejune wall opposite to that in which the anastomosis has to be performed.

Finally, the guide means (guide wires) are removed, for example by pulling the proximal end portions A' and B', as in the case where the locking members 61 are provided In other terms, the guide wires are pulled along the direction in which they can slide with respect to the anastomotic device. Optionally, a seal test of the anastomosis based on methylene blue can be provided.

In the case where the aforesaid method is part of a gastro-intestinal by-pass procedure, it is possible to proceed further to the performing of a further entero-enteroanastomosis, for example a jejuno-jejunoanastomosis or an ileus-jejuno anastomosis, optionally repeating the steps previously described.

Such as described in the more general form, or in the specific application to perform a gastro-jejunoanastomosis (G-J), the aforesaid method allows a continuous control both in the positioning step of the guide wires and in the steps of drawing together and connecting the tissue portions. The provided steps further allow an endoluminal approach for procedures until now applied in the conventional surgery or laparoscopically, even if laparoscopic or partially laparoscopic techniques are not excluded.

The above described method can further being used to perform different kinds of anastomoses, for example a jejuno-jejunoanastomosis, in particular to achieve a gastro-intestinal by-pass, or a colon-colon anastomosis with transanal access.

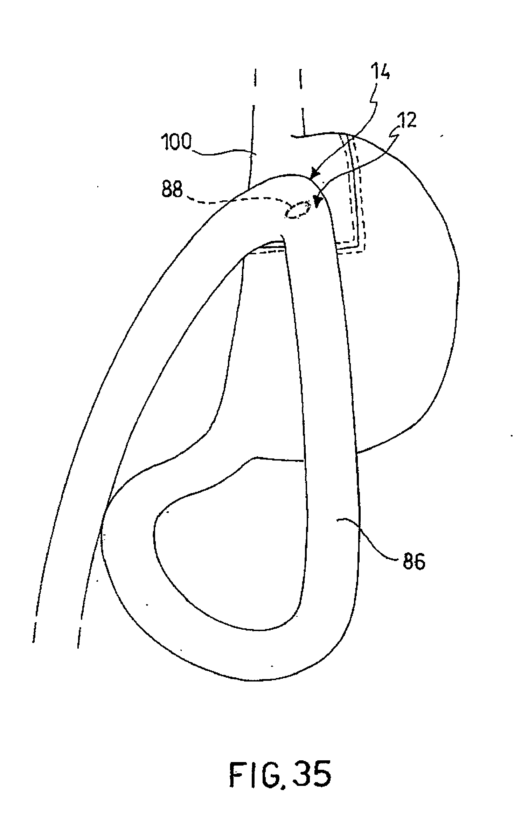

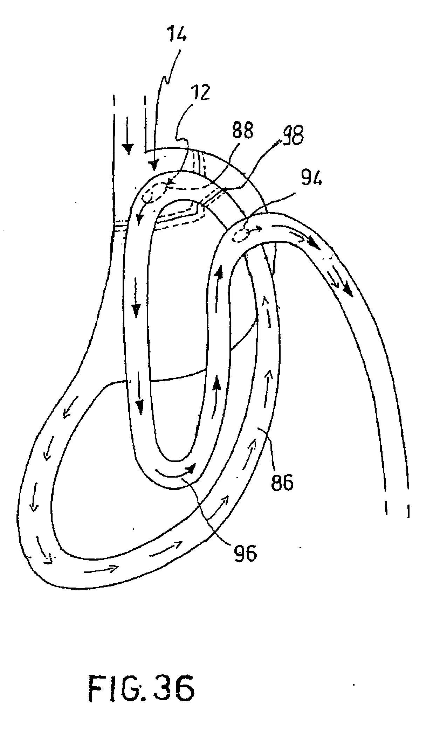

According to a possible embodiment, the method according to the present invention can be applied in a method for performing anastomoses 88, 94 in tracts of the digestive tube comprising the steps as illustrated in Figs. 35 and 36, in particular to obtain a gastro-intestinal by-pass. This method comprises drawing-together and connection steps of the tissues 12, 14 to form anastomoses adapted to keep or to reintegrate the integrity and the continuity of the intestinal duct after each formation of an anastomosis (both the gastro-jejuno anastomosis and the jejuno-jejuno anastomosis). Furthermore, the gastro-jejuno anastomosis and the jejuno-jejuno anastomosis are performed at a closed-up distance, allowing for a wide operative and visual field, while staying only in the upper area of the abdomen.

In this case also, the steps of drawing together tissues and creating the gastro-jejunoanastomosis and/or the jejuno-jejunoanastomosis can be performed using a guide means comprising at least two guide wires forming a loop passing through the tissue portions to be connected. The guide means can be associated to an anastomotic device as described above, or to other anastomotic or positioning devices. The aforesaid method can be performed endoluminally, or in a partially or wholly laparoscopic way.

In all the cases provided, the partial creation of a pocket 100 into the stomach, to which the first portion of the jejune will be connected can be preventively provided.

With reference to Figs. 35 and 36, subsequently a first loop is made of the guide wire through the open portion of the gastric pocket and through the portions of the jejune and the stomach to be connected. An anastomotic device is inserted and locked on the guide means and dragged, by the same guide means, until abutting against the first portion to be connected and drawing it together with the second portion to be connected. This first sequence of steps ends with the performing of a gastro-jejunoanastomosis 88 and the formation of a first loop 86 of intestine.

Subsequently, a second loop of guide wire is made through the open portion of the gastric pocket and through the two portions of the jejune to be connected. The path of the second loop can develop through the oesophagus and along the stomach and a tract of the intestine to one of the tissue portions to be connected; externally to the other tissue portion to be connected and from there along the intestine, through the gastro-jejunoanastomosis and the oesophagus. Or, both branches of the loop can pass through the gastro-jejunoanastomosis previously performed.

In this case also, an anastomotic device is inserted and locked in a direction on the guide means and dragged, by the same guide means, until abutting against the first portion to be connected and to drawing it together the second portion to be connected This second sequence of steps ends with the performing of an entero-enteroanastomosis 94, the carrying out of a second ring 96 of intestine and the completion of the gastric pocket 100. The cut performed between the two anastomoses has been indicated by 98.

The passing of the guide means through the walls of the tissues to be connected can be performed punching the wall (for example with radiofrequency needles) at the area intended to form the anastomosis, so as that after the formation of the anastomosis, the continuity of the intestinal duct is reintegrated.

An anastomotic device suitable for that aim can be the one described in the present application, or anastomotic or positioning devices suitable for releasing an anastomotic ring to perform the anastomosis, or a circular slidable stapler on the guide means and cooperating with an anvil, also slidable on the guide means.

The method previously described with reference to a procedure with guide means, preferably endoluminally, can be applied both to the performing step of the gastro-jejunoanastomosis and the performing step of the jejuno-jejunoanastomosis or to one of them.

This method this method allows to reduce the mortality risks in the case of gastro-intestinal bypasses and to considerably minimize the intervention times. The preservation of the continuity of the intestine until the completion of the two anastomoses allows for the simultaneous assessment of both. Furthermore, in virtue of the closed-up arrangement of the two anastomoses, the surgical field is restricted to the upper area of the abdomen.

To the preferred embodiment of the devices and methods above described, one skilled in the art, with the object to met contingent and specific needs, will be able to bring a number of modifications, adaptations and substitutions of elements with other functionally equivalent elements, without however departing from the scope of the following claims.

## Claims

1. An equipment to approximate tissue portions, which are intended to form an anastomosis, comprising:
a guide means suitable for passing through a first tissue portion (12) and a second tissue portion (14) to be connected by anastomosis,
an anastomotic device (10) to approximate the first tissue portion (12) and the second tissue portion (14) to be connected by anastomosis,
in which said guide means consists of at least two guide wires (A, B) suitable for passing through the tissue portions to be connected, to receive and to drag at least said anastomotic device (10) to approximate the first tissue portion (12) and the second tissue portion (14) to be connected by anastomosis.

2. The equipment to approximate tissue portions, which are intended to form an anastomosis according to claim 1, in which said guide wires (A, B) are located beside and are suitable for forming a loop which passes through the tissue portions to be connected by anastomosis and which extends between proximal end portions (A', B') and distal end portions (A", B").

3. The equipment to approximate tissue portions, which are intended to form an anastomosis according to claim 2, in which the distal end portions of said guide wires (A, B) are mutually connected and suitable for being split after the formation of said loop.

4. The equipment to approximate tissue portions, which are intended to form an anastomosis according to one of the preceding claims, in which at least one portion of the anastomotic device (10) is pre-associated to he guide wires so as it can be dragged by them in a direction and in such a way that the guide wires can be taken off by the anastomotic device in the opposite direction.

5. The equipment to approximate tissue portions, which are intended to form an anastomosis according to one of the preceding claims, comprising separation and identificative means of proximal end portions (A', B') and of distal end portions (A", B") of the guide wires (A, B).

6. The equipment to approximate tissue portions, which are intended to form an anastomosis according to claim 5, in which said separation and identificative means comprise a first sheath (74) suitable for being fitted on the proximal end portions (A', B') of the guide wires (A, B).

7. The equipment to approximate tissue portions, which are intended to form an anastomosis according to claim 6, in which said first sheath (74) has a characterizing feature.

8. The equipment to approximate tissue portions, which are intended to form an anastomosis according to one of claims 5 to 7, in which said separation and identificative means comprise a second sheath (84) suitable for being fitted on the distal end portions (A" , B") of the guide wires (A, B).

9. The equipment to approximate tissue portions, which are intended to form an anastomosis according to claim 8, in which said second sheath (84) has a characterizing feature.

10. The equipment to approximate tissue portions, which are intended to form an anastomosis according to claim 7 and 9, in which said separation and identificative means comprise said first sheath (74) and said second sheath (84) and in which the second sheath (84) has a characterizing feature different from that of the first sheath (74).

11. The equipment to approximate tissue portions, which are intended to form an anastomosis according to one of the preceding claims, in which said anastomotic device (10) comprises an abutment portion (18) suitable for the abutment against a surface of said first tissue portion (12) and a locking portion (20) suitable for being placed opposite the abutment portion (18) with respect to the first and second tissue portions (12, 14), said locking portion (20) and said abutment portion (18) being mutually connectable through the first and second tissue portions (12, 14) drawn together to keep them connected, at least one of said abutment portion (18) and said locking portion (20) being suitable for being connected to said guide means comprising at least two guide wires (A, B) to reach said first or second tissue portion (12, 14).

12. The equipment to approximate tissue portions, which are intended to form an anastomosis according to claim 11, in which said abutment portion (18) of said anastomotic device (10) is suitable for being locked in at least one direction on said guide wires (A, B) through which it is dragged until reaching the first tissue portion (12).

13. The equipment to approximate tissue portions, which are intended to form an anastomosis according to claim 12, in which said locking portion (20) is suitable for sliding along said at least two guide wires (A, B) and has a interaction portion with a positioning device (48) which biases them along said guide wires (A, B).

14. The equipment to approximate tissue portions, which are intended to form an anastomosis according to one of claims 11 to 13, in which at least one of said guide wires (A, B) passes through the abutment portion (18) and the locking portion (20) at connecting members between the two portions.

15. The equipment to approximate tissue portions, which are intended to form an anastomosis according to claim 14, in which at least one pin (22) extends from the abutment portion (18) and it is suitable for passing through said first tissue portion (12) and said second tissue portion (14) to be coupled to said locking portion (20) by being inserted in a respective housing (24) of the locking portion, said at least one pin (22) defining a channel (34) therein, which is suitable for receiving a guide wire (A, B) and in which the respective housing (24) of said locking portion (20) is suitable for housing said guide wire (A, B).

16. The equipment to approximate tissue portions, which are intended to form an anastomosis according to claim 15, in which said locking portion (20) is essentially ring-shaped.

17. Separation and identificative means of end portions of at least two guide wires (A, B) comprising a sheath suitable for being fitted on proximal (A', B') or distal end portions (A" , B") of the guide wires (A, B).

18. The separation and identificative means according to claim 32, comprising a first sheath (74) suitable for being fitted on the proximal end portions (A', B') of the guide wires (A, B).

19. The separation and identificative means according to claim 33, in which said first sheath (74) has a characterizing feature.

20. The separation and identificative means according to one of claims 32 to 34, comprising a second sheath (84) suitable for being fitted on the distal end portions (A", B") of the guide wires (A, B).

21. The separation and identificative means according to claim 35, in which said second sheath (84) has a characterizing feature.

22. The separation and identificative means according to claim 32 and 36, comprising said first sheath (74) and said second sheath (84) and in which the second sheath (84) has a characterizing feature different from that of the first sheath (74).
